(19) Europäisches Patentamt
European Patent Office
Office européen des brevets

(11) Veröffentlichungsnummer: **0 003 757**
B1

(12) **EUROPÄISCHE PATENTSCHRIFT**

(45) Veröffentlichungstag der Patentschrift: **14.10.81**

(51) Int. Cl.³: **C 07 D 209/66,** C 07 D 209/70, C 07 D 491/04

(21) Anmeldenummer: **79100271.0**

(22) Anmeldetag: **31.01.79**

(54) **Tricyclische Imidylderivate und Verfahren zu ihrer Herstellung.**

(30) Priorität: **08.02.78 CH 1400/78**

(43) Veröffentlichungstag der Anmeldung: **05.09.79 Patentblatt 79/18**

(45) Bekanntmachung des Hinweises auf die Patenterteilung: **14.10.81 Patentblatt 81/41**

(84) Benannte Vertragsstaaten: **CH DE FR GB IT NL SE**

(56) Entgegenhaltungen:
**DE-A-205 363**
**FR-A-1 418 511**
**US-A-3 542 805**
**US-A-3 941 746**

(73) Patentinhaber: **CIBA-GEIGY AG, Patentabteilung Postfach, CH-4002 Basel (CH)**

(72) Erfinder: **Zweifel, Hans, Dr., Lothringerstrasse 93, CH-4056 Basel (CH)**
Erfinder: **Schilling, Walter, Dr., Im Muspenacker, CH-4249 Himmelried (CH)**
Erfinder: **Storni, Angelo, Dr., Im Feuerbusch 3, CH-4310 Rheinfelden (CH)**
Erfinder: **Bellus, Daniel, Dr., Unterm Schellenberg 81, CH-4125 Riehen (CH)**

**EP 0 003 757 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Tricyclische Imidylderivate und Verfahren zu ihrer Herstellung

Die vorliegende Erfindung betrifft neue tricyclische Imidylderivate und Verfahren zu deren Herstellung. Die erfindungsgemäßen tricyclischen Imidylderivate eignen sich zur Herstellung von unter der Einwirkung von Licht vernetzbaren Polymeren.

Polycyclische Imide und deren Verwendung insbesondere als Kunststoffzusätze sind bekannt. So beschreibt die FR-Patentschrift FR-A-1 418 511 polyhalogenierte Polyhydro-methan-2,3-naphthalindicarbonsäureimide, die als Härter für Polymere eingesetzt werden können. Die US-Patentschrift US-A 3 542 805 offenbart ebenfalls endokondensierte polyhalogenierte polycyclische Imide, die u. a. als Flammschutzmittel in Kunststoffen, Harzen usw., als Zusatzstoffe für natürliche und synthetische Fette und Öle sowie als Insektizide Verendung finden. Aus der US-Patentschrift US-A-3 941 746 sind Norbornan-2,3-dicarbonsäureimide bekannt, welche am Stickstoff mit einem sterisch gehinderten Phenol- oder Hydroxyphenyl-Alkyl-Rest substituiert sind. Die Verbindungen werden als UV-Stabilisatoren für Polymere beschrieben.

Aus der Literatur ist ferner bekannt, daß sich verschiedenartig substituierte Imide, vor allem Maleinimide, zur Herstellung von vernetzbaren (härtbaren) Polymeren eignen. Die japanischen Offenlegungsschriften 50-5376, 50-5377, 50-5378, 50-5379 und 50-5380 beschreiben generisch verschiedene zur Herstellung von lichtvernetzbaren Polymeren geeignete α-Arylmaleinimide und N-substituierte Derivate davon, die in β-Stellung noch durch ein Halogenatom, eine Cyanogruppe oder eine niedere Alkylgruppe substituiert sein können, wobei die genannte Alkylgruppe zusammen mit dem ortho-ständigen C-Atom der α-Arylgruppe auch einen Ring bilden kann. Die konkrete Offenbarung beschränkt sich jedoch auf α-Phenyl- und α-Phenyl-β-cyanomaleinimide und N-substituierte Derivate davon. In den japanischen Offenlegungsschriften 49-128991, 49-128992, 49-128993, 50-9682, 50-10884 und 50-77363 wird die Herstellung von lichtvernetzbaren Polymeren, z. B. durch Umsetzung von N-substituierten α-Arylmaleinimiden der vorerwähnten Art, die am N-Substituenten Hydroxyl-, Amino-, Carbonsäure- oder Carbonsäurechloridgruppen aufweisen, mit Polymeren mit entsprechenden funktionellen Gruppen beschrieben. Aus den deutschen Offenlegungsschriften 2 031 573, 2 032 037 und 2 626 795 sind weitere Imidylderivate bzw. lichtvernetzbare Polymere mit end- oder seitenständigen Imidylgruppen, besonders Maleinimid-, Dimethylmaleinimid-, Nadicimid- und Tetrahydrophthalimidgruppen, bekannt.

Diese vorbekannten Imide bzw. die daraus herstellbaren vernetzbaren Polymeren haben den Nachteil einer relativ geringen photochemischen Empfindlichkeit, weshalb sie sich nicht oder nur schlecht für zahlreiche Anwendungen eignen, für die hochlichtempfindliche Substanzen benötigt werden, oder aber sie erfordern die Mitverwendung von bekannten Photosensibilisatoren, wie Benzophenon, Thioxanthon und dergleichen. Ferner eignen sich diese vorbekannten Imide zum Teil auch schlecht für den Aufbau von Polymeren durch Polymerisation oder Polykondensation von entsprechenden Monomeren.

Aufgabe der Erfindung war daher die Bereitstellung von neuen hoch lichtempfindlichen Substanzen, die eine hohe UV-Absorption aufweisen und deswegen auch ohne Zusatz von Photosensibilisatoren eine hohe Vernetzungsgeschwindigkeit gewährleisten und sich auch gut für den Aufbau von Polymeren durch Polymerisation oder Polykondensation, gegebenenfalls zusammen mit geeigneten Comonomeren, eignen.

Die neuen Verbindungen entsprechen der Formel I

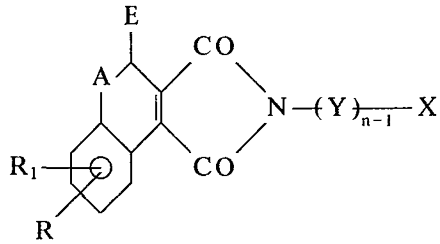

(I)

worin n die Zahl 1 oder 2, R und $R_1$ unabhängig voneinander Wasserstoff, Halogen, Alkyl mit 1–4 C-Atomen oder Methoxy, A $-CH_2-$, $-CH_2CH_2-$ oder $-OCH_2-$ mit an den aromatischen Ring gebundenem Sauerstoffatom und E Wasserstoff oder A $-O-$ und E $-CH_3$ und Y gegebenenfalls durch Heteroatome unterbrochenes Alkylen mit 1–30 C-Atomen, Cycloalkylen mit 5 oder 6 C-Atomen, einen Dicyclohexylmethanrest, Arylen mit 6–10 C-Atomen, Aralkylen oder Alkylarylen mit 7 oder 8 C-Atomen bedeuten, wobei die genannten Reste Y mit Alkyl- oder Alkoxygruppen mit je 1–4 C-Atomen, Nitrogruppen oder Halogenatomen substituiert sein können, X, bei n = 1, eine Gruppe der Formeln $-NH-CO$-Alkenyl oder

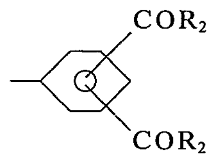 und,

bei $n = 2$, $-OH$, $-NH_2$, $-NH$-Alkyl mit 1–4 C-Atomen, $-SH$, $-COOH$, $-COCl$, $-CO-O$-Alkenyl, $-O$-Alkenyl, $-O-CO$-Alkenyl, $-NH-CO$-Alkenyl oder $-S-CO$-Alkenyl darstellt, die beiden $-COR_2$ in meta- oder para-Stellung zueinander an den Benzolring gebunden sind und die $R_2$ je $-OH$, $-Cl$, Alkoxy mit 1–4 C-Atomen oder Phenoxy bedeuten oder die beiden $-COR_2$ in ortho-Stellung zueinander an den Benzolring gebunden sind und eines von $R_2$ $-OH$ oder $-O^- M^+$ und das andere

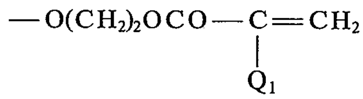

oder beide $R_2$ zusammen $-O-$ bedeuten, $M^+$ ein Alkalimetallkation, ein Pyridiniumkation oder ein Trialkylammoniumkation mit 3–24 und insbesondere 3–12 C-Atomen, $Q_1$ Wasserstoff oder Methyl und q eine ganze Zahl von 2–4 bedeuten, wobei in den obigen Gruppen Alkenylteile 2–4 C-Atome aufweisen und A bei $n = 2$ und $Y = -CH_2-$ einen von $-CH_2-$ verschiedenen Rest darstellt.

Von der Formel I werden Verbindungen der Formeln Ia und Ib

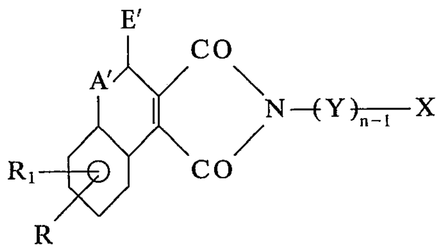 (Ia)

und

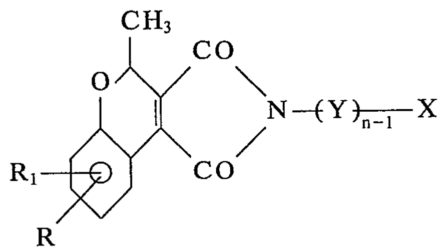 (Ib)

umfaßt, worin A' $-CH_2-$, $-CH_2CH_2-$ oder $-OCH_2-$ mit an den aromatischen Ring gebundenem Sauerstoffatom und E' Wasserstoff bedeuten, R, $R_1$, X, Y und n die unter Formel I angegebene Bedeutung haben und A' bei $n = 2$ und $Y = -CH_2-$ einen von $-CH_2-$ verschiedenen Rest darstellt.

Die Verbindungen der Formel Ia können dadurch hergestellt werden, daß man eine Verbindung der Formel II

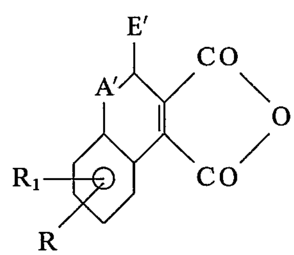 (II)

mit einer Verbindung der Formel III

$$H_2N-(Y)_{n-1}-X' \quad \text{(III)}$$

umsetzt, wobei A' und E' die unter Formel Ia angegebene und R, $R_1$, Y und n die unter Formel I

angegebene Bedeutung haben und X', bei $n = 1$, eine Gruppe der Formel

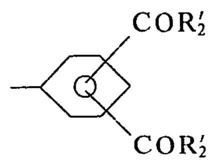

worin die $-COR_2'$-Gruppen in meta- oder para-Stellung zueinander an den Benzolring gebunden sind und die $R_2'$ je $-OH$, $-O^-M^+$, Alkoxy mit 1–4 C-Atomen oder Phenoxy darstellen und $M^+$ die unter Formel I angegebene Bedeutung hat, oder worin die $-COR_2'$-Gruppen in ortho-Stellung zueinander an den Benzolring gebunden sind und die beiden $R_2'$ zusammen $-O-$ darstellen, und, bei $n = 2$, $-OH$, $-NH_2$, $-NH$-Alkyl mit 1–4 C-Atomen, $-COOH$, $-SH$ oder $-O$-Alkenyl mit 2–4 C-Atomen im Alkylteil bedeutet, gegebenenfalls intermediär gebildete Amidcarbonsäuren cyclisiert und das Imid anschließend gegebenenfalls in eine Verbindung der Formel Ia überführt, worin X eine von X' verschiedene Bedeutung hat.

Die Verbindungen der Formel Ib können dadurch erhalten werden, daß man eine Verbindung der Formel I bzw. Ia, worin A $-OCH_2-$ mit an den aromatischen Ring gebundenem Sauerstoffatom darstellt und R, $R_1$, X, Y und n die unter Formel I angegebene Bedeutung haben, thermisch oder in Gegenwart eines basischen Katalysators in eine Verbindung der Formel Ib umlagert.

Sind die Brückenglieder Y definitionsgemäß mit Alkyl- oder Alkoxygruppen substituiert, so weisen diese Gruppen insbesondere 1 oder 2 C-Atome auf. Als Halogensubstituenten für die Brückenglieder Y kommen z. B. Chlor, Brom oder Fluor in Frage.

Alkylengruppen Y können geradkettig oder verzweigt sein und ein oder mehrere Heteroatome enthalten, besonders S- oder O-Atome. Bevorzugt sind unsubstituierte geradkettige oder verzweigte Alkylengruppen, besonders solche mit 2–11 C-Atomen. Beispiele von geeigneten Alkylengruppen Y sind die Äthylengruppe, die 1,3- oder Isopropylengruppe, die 2,2-Dimethylpropylengruppe, die Tetramethylen-, Hexamethylen-, Octamethylen- und Decamethylengruppe.

Stellt Y eine Cycloalkylengruppe dar, so ist diese bevorzugt unsubstituiert. Insbesondere handelt es sich um die 1,3- und vor allem die 1,4-Cyclohexylengruppe.

Sind Arylengruppen Y substituiert, so weisen diese Gruppen pro Ring bevorzugt nur einen Substituenten, insbesondere eine Alkyl- oder Alkoxygruppe mit je 1–4 und vor allem 1 oder 2 C-Atomen oder eine Nitrogruppe, auf. Beispiele geeigneter Arylengruppen Y sind die 1,2-, 1,3- und 1,4-Phenylengruppe, die 1,3-Tolylengruppe, die 5-Methoxy-1,3-phenylengruppe, die 3-Nitro-1,4-phenylengruppe, die 1,7- oder 2,7-Naphthylengruppe. Bevorzugt sind unsubstituierte Arylengruppen, besonders die 1,4- und die 1,3-Phenylengruppe.

Als Aralkylengruppen Y kommen vor allem die Gruppen

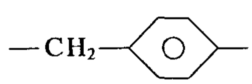 und 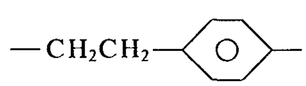

und als Alkylarylengruppen Y vor allem die Gruppen

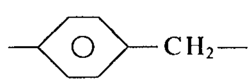 und 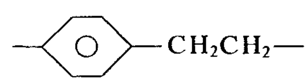

in Betracht.

Definitionsgemäße Alkyl- oder Alkoxygruppen R, $R_1$, $R_2$ oder $R_2'$ sowie Alkyl- oder Alkenylteile von definitionsgemäßen Substituenten X oder X' können ebenfalls geradkettig oder verzweigt sein.

Als Beispiele definitionsgemäßer Alkyl-, Alkoxy- und Alkenylgruppen seien genannt: die Methyl-, Äthyl-, Propyl-, Isopropyl-, n-Butyl-, tert.-Butyl-, Methoxy-, Äthoxy-, n-Propoxy-, Isopropoxy-, n-Butoxy-, tert.-Butoxy-, Vinyl-, Allyl- und Isopropenylgruppe.

Stellt R oder $R_1$ ein Halogenatom dar, so handelt es sich vor allem um Chlor-, Brom- oder Fluoratome. Alkylgruppen R und $R_1$ sind mit Vorteil geradkettig und weisen 1 oder 2 C-Atome auf. Bevorzugt stellen R und $R_1$ jedoch je Wasserstoff dar.

Alkoxygruppen $R_2$ bzw. $R_2'$ sind ebenfalls bevorzugt geradkettig und weisen 1 oder 2 C-Atome auf.

$M^+$ stellt beispielsweise das Lithium-, Natrium-, Kalium-, Trimethylammonium-, Triäthylammonium-, Methyldiäthylammoniumkation oder das Tri-n-octylammoniumkation dar. Bevorzugt bedeutet $M^+$ ein Alkalimetallkation, besonders das Natriumkation.

Unter den Gruppen

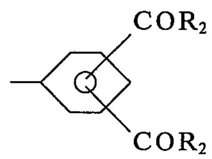

bzw.

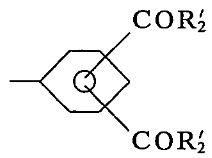

sind solche bevorzugt, worin die Gruppen $-COR_2$ bzw. $-COR_2'$ in meta-Stellung zueinander an den Benzolring gebunden sind und die $R_2$ bzw. $R_2'$ je $-OH$, Methoxy, Äthoxy, Phenoxy oder $-Cl$ darstellen, oder solche, worin die Gruppen $-COR_2$ bzw. $-COR_2'$ in ortho-Stellung zueinander an den Benzolring gebunden sind und die beiden $R_2$ bzw. $R_2'$ zusammen $-O-$ bedeuten.

Bevorzugt sind Verbindungen der Formel I bzw. Ia, worin R und $R_1$ je Wasserstoff, A $-CH_2-$, $-CH_2CH_2-$ oder $-OCH_2-$ mit an den aromatischen Ring gebundenem Sauerstoffatom, E Wasserstoff, Y geradkettiges oder verzweigtes Alkylen mit 2–11 C-Atomen, die 1,3- oder 1,4-Phenylengruppe oder die 1,4-Cyclohexylengruppe, X, bei n = 1, eine Gruppe der Formeln

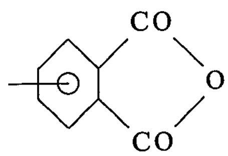 oder 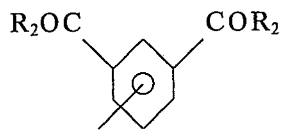

worin die beiden $R_2$ je $-OH$, $-Cl$, Methoxy, Äthoxy oder Phenoxy bedeuten, und, bei n = 2, $-OH$, $-NH_2$, $-COOH$, $-COCl$, $-COO$-Alkenyl, $-O$-Alkenyl oder $-O-CO$-Alkenyl darstellen und die Alkenylteile in den genannten Substituenten X 2–4 C-Atome aufweisen und insbesondere

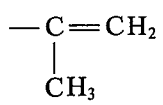 oder $-CH=CH_2$

darstellen.

Besonders bevorzugt sind Verbindungen der Formel I bzw. Ia, worin R, $R_1$, Y und X die oben angegebene bevorzugte Bedeutung haben, A $-CH_2-$ und E Wasserstoff darstellen.

Spezielle bevorzugte Verbindungen der Formel I sind Verbindungen der folgenden Formeln X bis XIV:

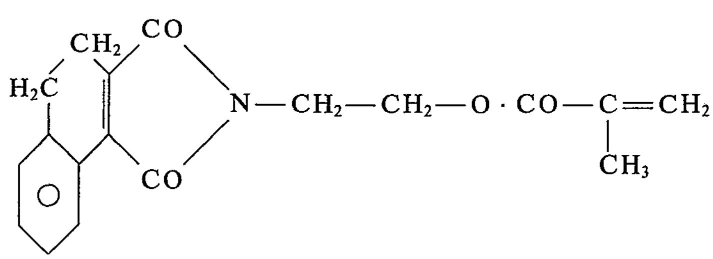

(X)

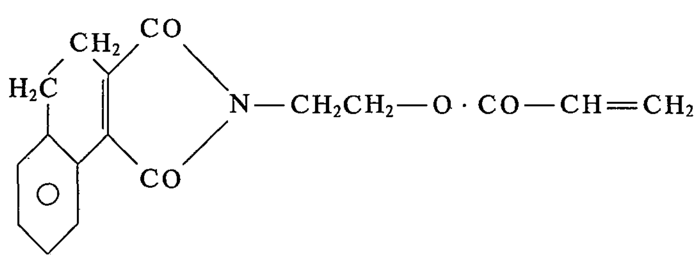

(XI)

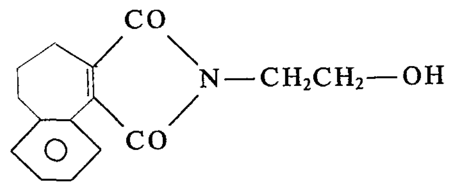

(XII)

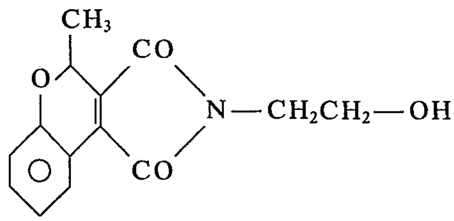

(XIII)

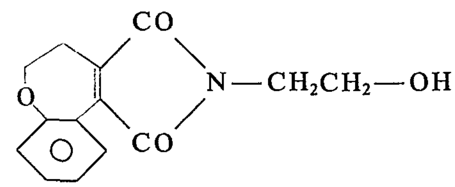

(XIV)

Die Verbindungen der Formel III und die Verbindungen der Formel II, worin A' $-CH_2-$ und E' Wasserstoff bedeuten, sind bekannt oder können nach an sich bekannten Methoden hergestellt werden. Die Verbindungen der Formel II, worin A' $-CH_2CH_2-$ oder $-OCH_2-$ mit an den aromatischen Ring gebundenem Sauerstoffatom und E' Wasserstoff darstellen, sind neu. Die neuen Verbindungen der Formel II, worin A' $-CH_2CH_2-$ und E' Wasserstoff bedeuten, können z. B. durch Umsetzung von gegebenenfalls ringsubstituierten 5-Phenylvaleriansäureestern, wie dem 5-Phenylvaleriansäureäthylester, mit einem Oxalsäurediester, z. B. dem Oxalsäurediäthylester, zum 3-Phenylpropyl-oxalessigsäurediester und Überführen des letzteren in das gegebenenfalls ringsubstituierte 6,7-Dihydro-5H-benzocyclohepten-8,9-dicarbonsäureanhydrid durch Behandeln mit einer starken Säure, wie konzentrierte Schwefelsäure, erhalten werden. Verbindungen der Formel II, worin A' $-OCH_2-$ mit an den aromatischen Ring gebundenem Sauerstoffatom und E' Wasserstoff bedeuten, lassen sich in analoger Weise durch Umsetzung von gegebenenfalls ringsubstituierten **Phenoxybuttersäureestern mit einem Oxalsäurediester und Behandeln des erhaltenen 2-Phenoxyäthyloxalessigsäurediesters mit einer starken Säure herstellen.**

Amine der Formel $H_2N-Y-O$-Alkenyl können z. B. durch Umsetzen entsprechender Aminoalkohole in Gegenwart von Basen, wie $K_2CO_3$, Triäthylamin oder Pyridin, mit Alkenylhalogeniden, besonders Alkenylbromide, erhalten werden.

Aminobenzoldicarbonsäuren und deren Derivate der Formel

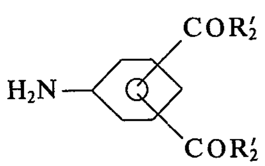

können als solche eingesetzt werden oder in situ durch Reduktion der entsprechenden Nitrobenzoldicarbonsäuren oder Derivaten davon hergestellt und ohne Zwischenisolierung weiterverwendet werden. Bevorzugt verwendet man die entsprechenden Ester und insbesondere die Salze, vor allem die Alkalimetallsalze.

Die Umsetzung der Amine der Formel III mit den Anhydriden der Formel II kann in der Schmelze durch Erhitzen der Reaktionspartner auf Temperaturen bis etwa 250° C, oder aber in wäßrigem, wäßrig-organischem oder organischem Medium vorgenommen werden, wobei je nach Reaktionskomponenten bei Temperaturen zwischen etwa 0° C und Siedetemperatur gearbeitet wird. Bevorzugt wird die Umsetzung in organischem Medium durchgeführt.

Zweckmäßig setzt man das Anhydrid der Formel II in stöchiometrischer Menge oder in einem leichten Überschuß über das Amin der Formel III ein, z. B. in einem bis zu etwa 20%igen molaren Überschuß.

Als organische Lösungsmittel kommen vor allem aprotische organische Lösungsmittel in Betracht. Beispiele derartiger Lösungsmittel sind: gegebenenfalls halogenierte aliphatische oder aromatische Kohlenwasserstoffe, wie Methylenchlorid, Dichlormethan, Chloroform, Tetrachlorkohlenstoff, 1,1,2-Trichloräthan, 1,2-Dichloräthylen, Benzol, Toluol und Chlorbenzol; wasserfreie Essigsäure;

cyclische Äther, wie Tetrahydrofuran, Tetrahydropyran und Dioxan; cyclische Amide, wie N-Methyl-2-pyrrolidon, N-Acetyl-2-pyrrolidon, N-Methylcaprolactam; N,N-Dialkylamide von aliphatischen Monocarbonsäuren mit 1–3 C-Atomen im Säureteil, wie N,N-Dimethylformamid, N,N-Dimethylacetamid, N,N-Diäthylacetamid, N,N-Dimethylmethoxyacetamid; N,N,N',N'-Tetramethylharnstoff; Tetrahydrothiophendioxid (Sulfolan); Dialkylsulfoxide, wie Dimethyl- und Diäthylsulfoxid.

Es können auch Gemische derartiger Lösungsmittel eingesetzt werden. Bevorzugte Lösungsmittel sind Dioxan, wasserfreie Essigsäure, Methylenchlorid, Benzol, Toluol, Xylole und Chlorbenzol.

Je nach Art der Reaktionskomponenten und der Reaktionsbedingungen, vor allem bei erhöhten Reaktionstemperaturen, lassen sich die Anhydride der Formel II mit den Aminen der Formel III direkt, d. h. ohne zusätzliche Maßnahmen, wie Behandeln mit Dehydratisierungsmitteln, zu den Imiden der Formel Ia umsetzen. Im allgemeinen entstehen jedoch intermediär Amidcarbonsäuren der Formel IV

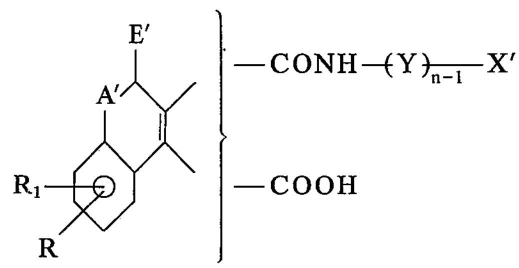

(IV)

worin n, R, $R_1$, Y, A', E' und X' die unter den Formeln I bzw. II und III angegebene Bedeutung haben. Diese Amidcarbonsäuren können auf an sich bekannte Weise chemisch oder thermisch zu den Imiden der Formel Ia cyclisiert werden, gegebenenfalls in Gegenwart eines aprotischen organischen Lösungsmittels der vorerwähnten Art.

Die thermische Cyclisierung kann z. B. durch Erhitzen des Reaktionsproduktes auf Temperaturen von etwa 50 bis 200° C vorgenommen werden. Bevorzugt ist jedoch die chemische Cyclisierung unter Verwendung an sich für die Imid- und gegebenenfalls die Anhydridbildung bekannter Dehydratisierungsmittel, gegebenenfalls in Gegenwart von Katalysatoren, bei Temperaturen zwischen etwa 40 und 150° C. Als Dehydratisierungsmittel kommen vor allem Anhydride von gegebenenfalls durch Halogenatome oder Alkylgruppen substituierten aliphatischen Monocarbonsäuren mit 2–5 C-Atomen in Betracht, wie Essigsäure-, Propionsäure-, Buttersäure- und Valeriansäureanhydrid, Trichlor- und Trifluoressigsäureanhydrid. Bevorzugtes Dehydratisierungsmittel ist Essigsäureanhydrid. Als Katalysatoren können bei der chemischen Cyclisierung beispielsweise Erdalkalimetall- oder Alkalimetallsalze von aliphatischen Monocarbonsäuren mit 1–3 C-Atomen, wie Natrium- und Kaliumacetat, mitverwendet werden.

Die erfindungsgemäß erhaltenen Verbindungen können gegebenenfalls anschließend nach an sich bekannten Methoden in Verbindung der Formel Ia übergeführt werden, worin X eine von X' verschiedene Bedeutung hat. Als Beispiele seien genannt:

1. $n=1$, $X = -NH-CO$-Alkenyl

Durch Umsetzung von Reaktionsprodukten, worin $n=1$ und $X = -NH_2$ mit Säurechloriden Alkenyl-COCl.

2. n = 1, X = 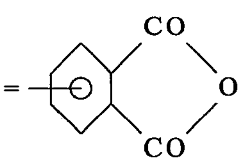

Durch Cyclisieren von Verbindungen der Formel I a, worin

n = 1 und X = 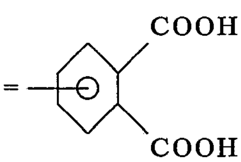

3. n = 1, X = 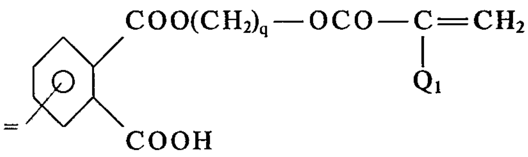

Durch Umsetzen von Verbindungen der Formel I a, worin n = 1 und X =

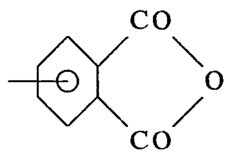

mit Alkoholen

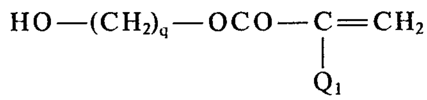

4. n = 1, X = 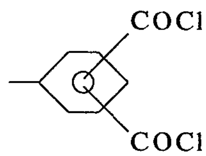 oder n = 2 und X = $-COCl$

Durch Umsetzen von Verbindungen der Formel I a, worin n = 1 und X =

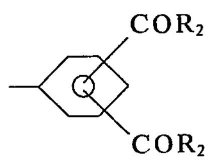

worin die Gruppen $-COR_2$ in meta- oder para-Stellung an den Benzolring gebunden sind und die beiden $R_2$ = je $-OH$ oder $-O^- M^+$ bzw. von Verbindungen der Formel Ia, worin n = 2 und X = $-COOH$, mit geeigneten Chlorierungsmitteln, wie Thionylchlorid, Oxalylchlorid oder Phosgen.

5. n = 2, X = $-CO-O$-Alkenyl

Durch Umsetzen von Verbindungen der Formel Ia, worin n = 2 und X = $-COOH$ oder $-COCl$ mit entsprechenden ungesättigten Estern oder Alkoholen in Gegenwart von Säuren oder Basen.

6. n = 2, X = $-O-CO$-Alkenyl oder $-S-CO$-Alkenyl

Durch Umsetzen von Verbindungen der Formel Ia, worin n = 2 und X = $-OH$ oder $-SH$ mit entsprechenden ungesättigten Säuren, Säurechloriden oder Estern.

7. n = 2, X = $-NH-CO$-Alkenyl

Durch Umsetzen von Verbindungen der Formel Ia, worin n = 2 und X = $-NH_2$ mit Säurechloriden Alkenyl-COCl.

8. n = 2, X = $-O$-Alkenyl

Durch Umsetzen von Verbindungen der Formel Ia, worin n = 2 und X = $-OH$ mit Alkenylhalogeniden, besonders Alkenylbromiden, in Gegenwart von Basen, wie $K_2CO_3$.

Verbindungen der Formel Ia, worin A $-OCH_2-$ mit an den aromatischen Ring gebundenem

Sauerstoffatom darstellt, können thermisch oder in Gegenwart eines basischen Katalysators in Verbindungen der Formel Ib umgelagert werden. Die Umlagerung wird zweckmäßig in einem organischen Lösungsmittel, beispielsweise einem aprotischen organischen Lösungsmittel der vorerwähnten Art oder in wasserfreier Essigsäure vorgenommen Die thermische Umlagerung erfolgt mit Vorteil durch Erhitzen des Reaktionsgemisches auf Temperaturen von etwa 80 bis 180°C, während etwa 6 bis 48 Stunden Für die thermische Umlagerung eignen sich insbesondere Verbindungen der Formel Ia, worin X eine nicht polymerisationsfähige Gruppe darstellt oder worin der Substituent X eine nicht polymerisationsfähige Gruppierung enthält.

Die Umlagerung in Gegenwart eines basischen Katalysators wird mit Vorteil bei Temperaturen zwischen etwa 60 und 130 C und insbesondere zwischen etwa 80 und 120°C vorgenommen. Für die katalytische Umlagerung eignen sich vor allem Verbindungen der Formel Ia, worin X eine polymerisationsfähige Gruppe darstellt oder eine derartige Gruppierung enthält. Als Basen kommen vor allem organische Basen in Betracht, besonders tertiäre Amine der Formel

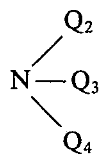

worin $Q_2$ Alkyl mit 1–8 C-Atomen, Cycloalkyl mit 5 oder 6 C-Atomen, Benzyl oder Phenyl und $Q_3$ und $Q_4$ unabhängig voneinander Alkyl mit 1–8 C-Atomen bedeuten, beispielsweise Triäthylamin, Tri-n-butylamin, Tri-isopentylamin, Tri-n-octylamin, N,N-Dimethyl-cyclohexylamin, N,N-Dimethylbenzylamin, N,N-Dimethyl-2-äthylhexylamin, N,N-Diäthylanilin; tertiäre cyclische Amine, z. B. N-Alkylmorpholine, wie N-Methylmorpholin; N-Alkylpiperidine, wie N-Methyl- und N-Äthylpiperidin; N-Alkylpyrrolidine, wie N-Methyl- und N-Äthylpyrrolidin; Chinuclidin und Diazabicyclo[2.2.2]octan; tertiäre Diamine, wie N,N,N',N'-Tetramethyläthylendiamin, N,N,N',N'-Tetramethyl-1,3-diaminobutan und N,N'-Dimethylpiperazin; ferner bicyclische Amidine, wie 1,5-Diazabicyclo[5.4.0]undec-5-en, und schließlich polymere basische Verbindungen, wie p-Dimethylaminomethylpolystyrol.

Die Menge der eingesetzten Katalysatoren kann innerhalb breiter Grenzen variieren. In manchen Fällen genügt es, wenn der Katalysator in Spuren vorliegt. Im allgemeinen wird jedoch der Katalysator bevorzugt in einer Menge von etwa 0,1 bis 15 Gew.-%, bezogen auf die Ausgangsverbindungen der Formel Ia mit $A = -OCH_2-$, eingesetzt.

Nach Beendigung der Umsetzung können die Verbindungen der Formel I auf übliche Weise isoliert und gegebenenfalls gereinigt werden.

Die Verbindungen der Formel I stellen wertvolle Zwischenprodukte zur Herstellung von lichtvernetzbaren Polymeren dar, wie Polyester, Polyamide, Polyimide, Polyester-Polyamide, Polyäther, Polyamine, Gelatine, Polysaccharide, Polykondensate, z. B. auf der Basis von Phenol-Formaldehyd, und Homo- und Copolymere, die sich von Monomeren mit reaktiven C=C-Doppelbindungen ableiten. Derartige Polymere lassen sich nach bekannten Synthesemethoden zur Herstellung von Makromolekülen mit photoaktiven seitenständigen Gruppen erhalten. Grundsätzlich kommen zwei Methoden in Betracht:

1. Einbau von Verbindungen der Formel I in eine bestehende Polymerkette mit entsprechenden funktionellen Gruppen. Für dieses Verfahren eignen sich z. B. Verbindungen der Formel I, worin X $-OH$, $-NH_2$, $-NH$-Alkyl mit 1–4 C-Atomen, $-SH$, $-COOH$ oder $-COCl$ darstellt oder worin ortho-ständige $R_2$ zusammen $-O-$ bilden. Derartige Verbindungen lassen sich beispielsweise mit Polymeren mit $NH_2$-, NH-Alkyl-, OH-, COOH-, SH-, Anhydrid- oder

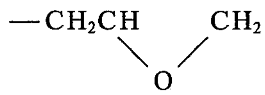

Gruppen und mit Phenoxyharzen mit seitenständigen OH-Gruppen umsetzen.

2. Aufbau der Polymerkette aus Verbindungen der Formel I und gegebenenfalls weiteren Monomeren, wobei die Polymerkette je nach Art der funktionellen Gruppen in der Verbindung der Formel I durch Polymerisation oder Polykondensation aufgebaut werden kann. Für dieses Verfahren eignen sich beispielsweise Verbindungen der Formel I, worin X bei $n = 1$ die angegebene Bedeutung hat oder Verbindungen der Formel II, worin X bei $n = 2$ $-CO-O$-Alkenyl, $-O$-Alkenyl, $-O-CO$-Alkenyl, $-NH-CO$-Alkenyl oder $-S-CO$-Alkenyl bedeutet. Es können auch Verbindungen der Formel I, worin die $-COR_2$ in meta- oder para-Stellung zueinander an den Benzolring gebunden sind und die $R_2$ je $-OH$, $-Cl$, Alkoxy mit 1–4 C-Atomen oder Phenoxy bedeuten, oder worin die $-COR_2$ in ortho-Stellung zueinander an den Benzolring gebunden sind und die $R_2$ zusammen $-O-$ darstellen, mit Diaminen, Diolen, Aminoalkoholen und gegebenenfalls weiteren Di-, Tri- oder Tetracarbonsäurederivaten polykondensiert werden.

Verbindungen der Formel I, worin X eine polymerisationsfähige Gruppe darstellt oder eine solche

Gruppierung enthält, eignen sich zur Homopolymerisation oder Copolymerisation mit anderen äthylenisch ungesättigten Comonomeren, z. B. Vinylchlorid, Vinylidenchlorid, Acrylsäure, Methacrylsäure, Acrylnitril, Methacrylnitril, Acrylsäure- und Methacrylsäurealkylester, Acrylamid, Methacryl amid, Styrol, Vinylpyridine, Äthylen, Propylen, Essigsäure- und Propionsäurevinylester, Malein- oder Fumarsäuresäureester oder Maleinsäureanhydrid.

Die so erhaltenen Polymeren mit seitenständigen Imidylgruppen lassen sich unter der Einwirkung von Licht, besonders UV-Licht, vernetzen und eignen sich für photomechanische Anwendungen, beispielsweise zur Herstellung von Druckplatten für das Offsetdruckverfahren, zur Herstellung von Photooffset-Lacken, für die unkonventionelle Photographie, z. B. zum Anfärben von nach dem Belichten und Entwickeln schlecht sichtbaren Polymerbildern mit geeigneten Farbstoffen, wie öllösliche Farbstoffe oder, wenn das Polymere saure Gruppen, wie Carbonsäure- oder Sulfonsäuregruppen, aufweist, kationische Farbstoffe. Solche Polymere finden insbesondere Anwendung als sogenannte Photoresist zur Herstellung von gedruckten Schaltungen nach an sich bekannten Methoden. Dabei wird die mit der lichtempfindlichen Schicht versehene Seite der Leiterplatte durch ein das Leiterbild aufweisendes Dianegativ belichtet und dann entwickelt, worauf man die unbelichteten Stellen der Schicht durch Entwicklungsflüssigkeit herausholt. Die Belichtung kann mit Sonnenlicht, Kohlelichtbogen oder Xenonlampen durchgeführt werden. Mit Vorteil wird die Belichtung mit Quecksilberhochdrucklampen vorgenommen.

Beispiel 1

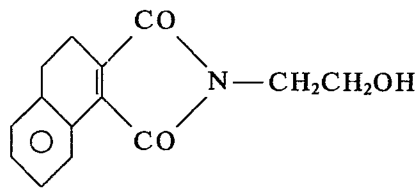

Eine Lösung von 70 g (0,35 Mol) 3,4-Dihydronaphthalin-1,2-dicarbonsäureanhydrid [hergestellt nach Org. Syntheses, Col. Vol., 2, 194 (1943)] und 23,5 g (0,385 Mol) Äthanolamin in 1,7 Liter Eisessig wird 24 Stunden unter Rückfluß gehalten. Danach wird der Eisessig destillativ entfernt, der Rückstand wird in 2 Liter absolutem Äthanol gelöst, 50 g eines Ionenaustauschers [»Dowex 50 W«, der Fa. Fluka AG] werden zugegeben, und die Suspension wird 24 Stunden unter Rückfluß gehalten. Anschließend wird der Ionenaustauscher abfiltriert, der Äthanol wird abdestilliert, und der Rückstand wird aus Diäthyläther/Äthanol umkristallisiert. Man erhält 61,8 g (73% d. Th.) N-(2'-Hydroxyäthyl)-3,4-dihydronaphthalin-1,2-dicarboximid; Smp. 120,5—121°C.

IR-Spektrum ($CHCl_3$): u. a. 2,93; 5,67; 5,88; 7,0; 7,18; 7,36; 7,61; 9,30; 9,90 μ.

NMR-Spektrum ($CDCl_3$): $\delta = 2,7$ und 3,02 [2xt, 2x2H, $H_2$—C(3) und $H_2$—C(4)]: 3,6 (bs, 4H, 2x$CH_2$ in der Hydroxyäthylgruppe); 7,1—7,4 [m, 3H, H—C(5), H—C(6), H—C(7)]; 8,0—8,2 ppm [m, 1H, H—C(8)].

UV-Spektrum ($C_2H_5OH$): $\lambda_{max}$ ($\varepsilon$) = 247 (12 960) und 367 (2 670) nm.

Elementaranalyse für $C_{14}H_{15}NO_4$ (Molgewicht 243,27):

| | | | |
|---|---|---|---|
| berechnet | C 69,12% | H 5,38% | N 5,76% |
| gefunden | C 68,95% | H 5,33% | N 5,74%. |

Wird im obigen Beispiel der Rückstand nach dem Abdestillieren des Eisessigs an einer Kieselgelsäule chromatographisch getrennt, so gewinnt man außer der gewünschten N-Hydroxyäthylverbindung auch deren Acetat; 103—105°C (aus Diäthyläther/n-Hexan umkristallisiert).

Beispiel 2

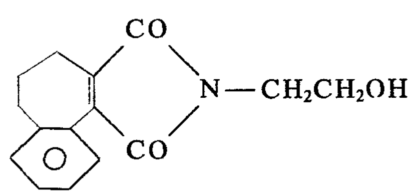

a) Zu einer Lösung von 15 g (0,07 Mol) 6,7-Dihydro-5H-benzocyclohepten-8,9-dicarbonsäureanhydrid in 150 ml Toluol gibt man bei Raumtemperatur (20—25°C) 4,5 g Äthanolamin zu. Während 2 Stunden wird unter Rückfluß gekocht, und das gebildete Wasser wird kontinuierlich mit einem Wasserabscheider entfernt. Nachher wird das Toluol destillativ entfernt, und der Rückstand wird aus Äthanol umkristallisiert. Man erhält 1,4 g (82% d. Th.) gelber Kristalle mit Smp. 115°C.

IR-Spektrum ($CHCl_3$): u. a. 2,92; 5,67; 5,87; 6,98; 7,12; und 7,36 $cm^{-1}$.

Elementaranalyse für $C_{15}H_{15}NO_3$ (Molgewicht 257,29):

| | | | |
|---|---|---|---|
| berechnet | C 70,02% | H 5,88% | N 5,44% |
| gefunden | C 70,11% | H 5,91% | N 5,60%. |

In den folgenden Abschnitten b), c) und d) wird die Herstellung des 6,7-Dihydro-5H-benzocyclohepten-8,9-dicarbonsäureanhydride beschrieben:

b) 5-Phenylvaleriansäureäthylester

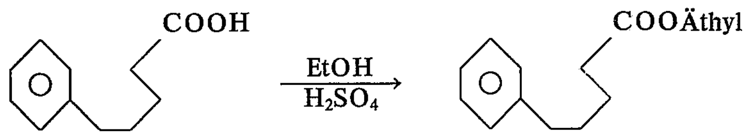


(A) (B)

250 g (1,4 Mol) 5-Phenylvaleriansäure löst man in 450 ml absolutem Äthanol. Zur klaren, farblosen Lösung gibt man 114 ml konzentrierte Schwefelsäure und erhitzt das Reaktionsgemisch während 48 Stunden am Rückfluß. Das zunächst zweiphasige Reaktionsgut wird annähernd homogen und trennt sich beim Kühlen wider in zwei Phasen. Das kalte zweiphasige Reaktionsgut wird aus Diäthyläther und ca. 1 kg Eis gegossen. Die Wasserphase wird noch 2mal ausgeäthert; die Ätherphasen wäscht man 2mal mit 2N-Sodalösung und 2mal mit NaCl-Lösung. Die vereinigten Ätherphasen trocknet man über $MgSO_4$ und entfernt das Lösungsmittel am Rotationsverdampfer. Nach dem Trocknen im Hochvakuum bei Raumtemperatur erhält man 281,7 g eines farblosen Öls (97,5% d. Th.).

Das Rohprodukt wird weiterverwendet (vgl. Abschnitt c). Zur Charakterisierung verwendet man eine bei 120—140°C/0,1 Torr im Kugelrohr destillierte Probe.

NMR-Spektrum ($CDCl_3$): =7,4—7,0 ppm (5H, m); 4,08 ppm (q, 2H, J=8 Hz); 2,6 ppm (m, 2H); 2,28 ppm (m, 2H); 1,65 ppm (2H, m); 1,02 ppm (3H, t, J=8 Hz).

IR-Spektrum ($CH_2Cl_2$): u. a. 1740 $cm^{-1}$.

c) 3-Phenylpropyl-oxalessigsäure-diäthylester

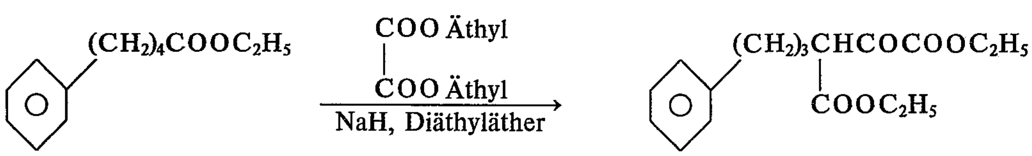


(B) (C)

Eine Suspension von ölfreiem Natriumhydrid in Diäthyläther, hergestellt durch zweimaliges Waschen von 71,8 g einer Natriumhydrid-Dispersion (55%ig, in Öl) mit n-Pentan unter Stickstoff, und 3 Liter absoluter Diäthyläther werden zum Rückfluß gebracht. Zur siedenden Suspension gibt man tropfenweise ein Gemisch von 281,7 g (1,36 Mol) 5-Phenylvaleriansäureäthylester und 297 g (1,36 m+50%) Oxalsäurediäthylester während ca. 6 Stunden. Das Reaktionsgut wird anschließend total während 66 Std. am Rückfluß gehalten. Im Dünnschichtchromatogramm ($CHCl_3$) ist neben sehr wenig Ausgangsmaterial ($R_F$ ca. 0,6) ein Hauptfleck mit $R_F$ ca. 0,5 vorhanden. Das Reaktionsgut gießt man nach dem Erkalten auf 500 g Eis und 1,05 Äquivalente HCl (=530 ml 2nHCl). Die Wasserphase extrahiert man mit Diäthyläther, trocknet den Diäthyläther über $MgSO_4$ und entfernt ihn im Vakuum. Nach dem Trocknen im Vakuum erhält man 520 g rötliches Öl, das noch Oxalester enthält. Das Rohprodukt wird direkt weiterverwendet, da es bei der destillativen Reinigung unter Decarbonylierung zerfällt.

Im NMR-Spektrum des Rohproduktes sind neben den Signalen des gewünschten Produktes noch die Signale des überschüssigen Oxalsäurediäthylesters sichtbar.

d) 6,7-Dihydro-5H-benzocyclohepten-8,9-dicarbonsäureanhydrid

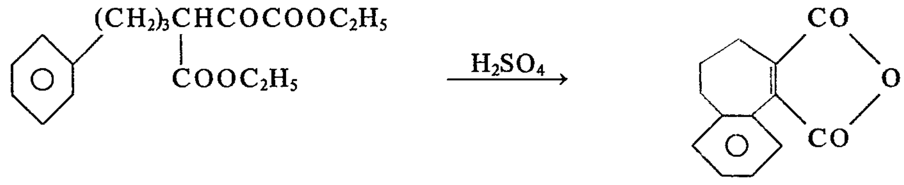


(C) (D)

240 ml 90%ige Schwefelsäure werden auf 0—5° C gekühlt. Bei dieser Temperatur tropft man innerhalb von ca. 15—20 Min. 30 g des Esters C zu. Es entsteht eine dunkelgelbe bis rötliche Lösung. Das Reaktionsgut läßt man sodann auf Raumtemperatur aufwärmen und verfolgt den Fortgang der Reaktion mittels Dünnschichtchromatographie. Nach ca. 3 bis 4 Std. ist kein Ausgangsmaterial mehr sichtbar.

Dünnschichtchromatogramm ($CHCl_3$) Ausgangsmaterial: $R_F$ ca. 0,7; Reaktionsprodukt: $R_F$ ca. 0,8.

Das Reaktionsgut gießt man auf 1,5 Liter Eis und genügend NaCl zur Sättigung der entstehenden Wasserphase (ca. 500 g). Unter heftigem Rühren scheidet sich ein weißer, kristalliner Niederschlag ab. Dieser wird abgenütscht, scharf abgesaugt, in Diäthyläther aufgenommen und von unlöslichen Bestandteilen abgetrennt. Die Ätherlösung wird über $MgSO_4$ getrocknet, am Rotationsverdampfer eingeengt und am Hochvakuum getrocknet. (= 1 Teil von Produkt D.)

Die Wasserphase wird mit Diäthyläther ausgezogen, der Diäthyläther wird mit NaCl-Lösung gewaschen, getrocknet und am Rotationsverdampfer entfernt. (= 2. Teil von Produkt D.) Auf Grund des Dünnschichtchromatogramms praktisch identisch mit dem ersten Teil.

Die beiden Teile werden vereinigt und aus Isopropanol umkristallisiert. Man erhält 10 g (47% d. Th.) der Verbindung D in Form schwach gelblicher Kristalle; Smp. 112—113° C.

## Beispiel 3

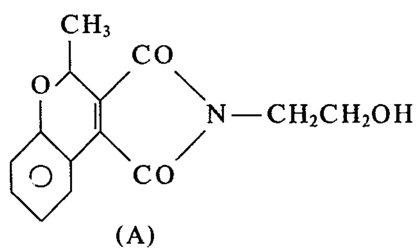

und

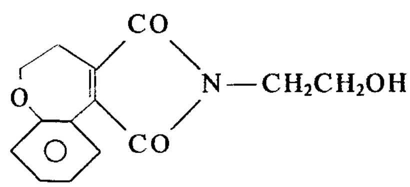

(A) (B)

Eine Lösung von 48,65 g (0,2 Mol) 2,3-Dihydro-1-benzoxepin-4,5-dicarbonsäureanhydrid und 13,4 g (0,22 Mol) Äthanolamin in 1,5 Liter Eisessig wird 2 Tage unter Rückfluß gehalten. Danach wird der Eisessig abdestilliert, der Rückstand wird in 2 Liter absolutem Methanol gelöst, 70 g eines Ionenaustauschers (»Dowex 50 W« der Fa. Fluka AG) werden zugegeben, und die Suspension wird 2 Tage unter Rückfluß gehalten. Anschließend wird der Ionenaustauscher abfiltriert, das Methanol wird destillativ entfernt, und der Rückstand wird an einer Kieselgelsäule (Fließmittel Toluol/Äthylacetat im Volumenverhältnis 2 : 1) getrennt. Die erste Fraktion ($R_F$ ca. 0,3) enthält 9,3 g (18% d. Th.) N-(2'-Hydroxyäthyl)-2-methyl-2H-chromen-3,4-dicarboximid (Verbindung A) in Form gelber Kristalle (aus Methylenchlorid/n-Hexan umkristallisiert); Smp. 124° C.

IR-Spektrum (KBr): u. a. 2,88; 5,65; 5,83; 6,21; 6,36; 6,90; 7,17; 9,53; 10,0; 13,14 μ.

NMR-Spektrum ($CDCl_3$): $\delta$ = 1,67 [d; J = 6,5; $CH_3C(2)$]; 3,78 (b"s"; 4H; 2x$CH_2$ in der Hydroxyäthylgruppe); 5,46 [t, J = 6,5; H—C(2)] und 6,8—7,1 + 7,2—7,4 + 7,94 ppm (m + m + d × d; 2H + 1H + 1H; aromat. H).

UV-Spektrum ($C_2H_5OH$): $\lambda_{max}$ ($\varepsilon$) = 256 (13 370) und 404 (2 920) nm.

Elementaranalyse für $C_{14}H_{13}NO_4$ (Molgewicht 259,27):

| | | | |
|---|---|---|---|
| berechnet | C 64,86% | H 5,05% | N 5,40% |
| gefunden | C 64,81% | H 5,11% | N 5,40%. |

Die zweite Fraktion ($R_f$ ca 0,2) enthält 41,5 g (80% d. Th.) N-(2'-Hydroxyäthyl)-2,3-dihydro-1-benzoxepin-4,5-dicarboximid (Verbindung B), Smp. 136–137 °C (aus $CH_2Cl_2$/n-Hexan umkristallisiert)

IR-Spektrum (KBr): 2,86; 5,64; 5,86; 6,90; 7,06; 7,62; 9,90; 13,3 und 13,9 μ.

NMR-Spektrum ($CDCl_3$): $\delta = 3{,}03$ [t; J = 5; $H_2$—C(3)]; 3,8 (b"s"; 4H; 2 × $CH_2$ in der Hydroxyäthylgruppe); 4,28 [t; $H_2$—C(2)] und 7,0—7,5 + 8,67 ppm (m + d × d; 3H + 1H; aromat. H).

UV-Spektrum ($C_2H_5OH$): $\lambda_{max}$ ($\varepsilon$) = 248 (12 820), 268 (7 900) und 354 (4 120) nm.

Elementaranalyse für $C_{14}H_{13}NO_4$ (Molgewicht 259,27):

| | | | |
|---|---|---|---|
| berechnet | C 64,86% | H 5,05% | N 5,40% |
| gefunden | C 64,76% | H 5,10% | N 5,32%. |

Das Ausgangsprodukt (2,4-Dihydro-1-benzoxepin-4,5-dicarbonsäureanhydrid) kann wie folgt hergestellt werden:

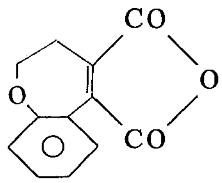

Zu einer eisgekühlten Mischung von 225 ml konz. Schwefelsäure und 25 ml Wasser läßt man unter Rühren 30,8 g (0,10 Mol) rohen 2-Phenoxyäthyloxalessigsäure-diäthylester innerhalb von 15 Minuten bei einer Temperatur von 5—10 °C zutropfen. Anschließend läßt man die Reaktionstemperatur auf 15 °C ansteigen und rührt eine Stunde bei dieser Temperatur. Hierauf gießt man das Reaktionsgemisch unter Rühren zu einer Mischung von 1000 g Eis und 1500 ml Wasser, wobei das 2,3-Dihydro-1-benzoxepin-4,5-dicarbonsäureanhydrid ausfällt. Dieses wird abgenutscht und aus Isopropanol umkristallisiert. Man erhält 14,0 g 2,3-Dihydro-1-benzoxepin-4,5-dicarbonsäureanhydrid (64,7% d. Th.) vom Smp. 142—143 °C.

Das Ausgangsprodukt (2-Phenoxyäthyl-oxalessigsäure-diäthylester) für die Herstellung des obengenannten Anhydrids kann wie folgt hergestellt werden:

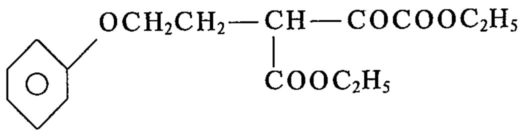

Zu einer Suspension von 5 g (0,104 Mol) einer 50%igen Natriumhydrid-Dispersion in Mineralöl in 50 ml Diäthyläther tropft man unter Rühren bei einer Temperatur von 15 °C eine Lösung von 22 g (0,15 Mol) Oxalsäure-diäthylester in 100 ml Diäthyläther zu. Anschließend läßt man zwei Stunden bei Raumtemperatur rühren. Dazu läßt man eine Lösung von 21 g (0,10 Mol) Phenoxy-buttersäure-äthylester [hergestellt nach Powell und Adams, J. Amer. Chem. Soc., 42, 652 (1920)] in 100 ml Diäthyläther zulaufen und kocht anschließend während 10 Stunden unter Rückfluß. Nach dem Abkühlen fügt man 1 ml Äthanol hinzu und gießt dann auf eine Mischung von 100 g Eis und 150 ml Wasser. Die wäßrige Phase wird mit 2N Salzsäure auf pH 3 gestellt. Im Scheidetrichter werden die Schichten getrennt, und die wäßrige Phase wird nochmals mit 250 ml Diäthyläther extrahiert. Die vereinigten Ätherextrakte werden mit 100 ml Wasser gewaschen, dann über Magnesiumsulfat getrocknet und anschließend im Wasserstrahlvakuum eingedampft. Als Rückstand verbleiben 30,8 g (100% d. Th.) roher 2-Phenoxyäthyl-oxalessigsäure-diäthylester in Form eines schwach rötlichen Öls.

## Beispiel 4

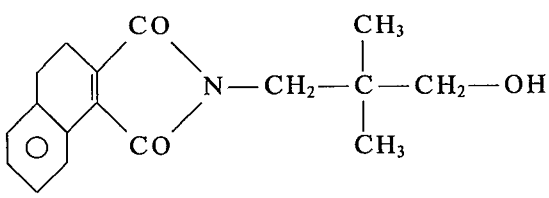

20,0 g (0,1 Mol) 3,4-Dihydronaphthalin-1,2-dicarbonsäureanhydrid und 10,3 g (0,1 Mol) 2,2-Dimethyl-3-aminopropanol werden in 60 ml Toluol gelöst und während 2 Stunden am Rückfluß gekocht, wobei

das entstehende Wasser durch einen Wasserabscheider abgetrennt wird. Nach beendeter Reaktion wird am Vakuum bei 60 C zur Trockne eingeengt. Der Rückstand wird aus Äthanol umkristallisiert. Man erhält 25,1 g (87,9% d. Th.) N-(3'-Hydroxy-2',2'-dimethylpropyl)-3,4-dihydronaphthalin-1,2 dicarbonsäureimid.

Elementaranalyse für $C_{17}H_{19}O_3N$ (Molgewicht 285):

| | | | |
|---|---|---|---|
| berechnet | C 71,56% | H 6,71% | N 4,91% |
| gefunden | C 70,74% | H 6,93% | N 4,75%. |

Beispiel 5

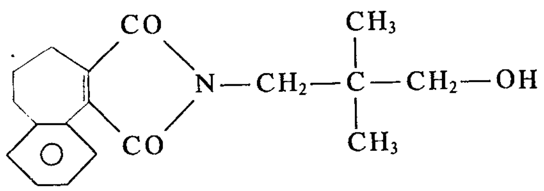

21,4 g (0,1 Mol) 6,7-Dihydro-5H-benzocyclohepten-8,0-dicarbonsäureanhydrid und 10,3 g (0,1 Mol) 2,2-Dimethyl-3-aminopropanol werden in 60 ml Toluol gelöst und während 1 Stunde am Rückfluß gekocht, wobei das entstehende Wasser durch einen nachgeschalteten Wasserabscheider abgetrennt wird. Nach dem Abkühlen auf Raumtemperatur werden die ausgefallenen Kristalle abfiltriert. Man erhält 28,9 g (96,6% d. Th.) N-(3'-Hydroxy-2',2'-dimethylpropyl)-6,7 dihydro 5H benzo cyclohepten-8,9-dicarbonsäureimid

Elementaranalyse für $C_{18}H_{21}O_3N$ (Molgewicht 299):

| | | | |
|---|---|---|---|
| berechnet | C 72,22% | H 7,07% | N 4,68% |
| gefunden | C 72,07% | H 7,12% | N 4,73%. |

Beispiel 6

24,3 g (0,1 Mol) des gemäß Beispiel 1 erhaltenen N-(2'-Hydroxyäthyl)-3,4-dihydronaphthalin-1,2-dicarbonsäureimids, 12,9 g (0,15 Mol) Methacrylsäure, 1,6 ml konz. Schwefelsäure und 1,0 g 2,6-Di-tert-butyl-p-kresol werden in Toluol gelöst und während 2 Stunden am Rückfluß gekocht, wobei das entstehende Wasser durch einen nachgeschalteten Wasserabscheider abgetrennt wird. Anschließend wird auf Raumtemperatur abgekühlt, die Reaktionslösung wird mit 5,52 g (0,075 Mol) Calciumhydroxid versetzt und während 5 Minuten ausgerührt. Nach dem Abfiltrieren wird das Filtrat am Vakuum bei 60°C zur Trockne eingeengt. Man erhält 28,8 g (95,7% d. Th.) N-(2'-Methacryloyloxyäthyl)-3,4-dihydronaphthalin-1,2-dicarbonsäureimid.

NMR-Spektrum: $H_2C=C$-Protonen bei 5,8 und 6,05 ppm (TMS = O).

Elementaranalyse für $C_{18}H_{17}NO_4$ (Molgewicht 311,33):

| | | | |
|---|---|---|---|
| berechnet | C 69,45% | H 5,46% | N 4,50% |
| gefunden | C 68,65% | H 5,42% | N 4,67%. |

Beispiel 7

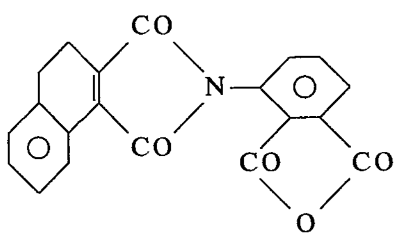

22,3 g (0,1 Mol) 3-Aminophthalsäure-dinatriumsalz und 20,0 g (0,1 Mol) 3,4-Dihydronaphthalin-1,2-dicarbonsäureanhydrid werden in einer Reibschale gut vermischt und dann während 2 Stunden bei 140°C gehalten. Anschließend wird während einer Stunde auf 160°C aufgeheizt. Nach dem Abkühlen auf Raumtemperatur wird die feste Masse pulverisiert, bei 90°C in 750 ml Wasser gelöst und nach dem

Abkühlen auf ca. 40°C mit 220 ml 1 N HCl angesäuert. Der resultierende Niederschlag wird abfiltriert und am Vakuum bei 60°C getrocknet. Das getrocknete Produkt wird anschließend in 750 ml Essigsäureanhydrid gelöst und am Rotationsverdampfer bei 70°C zur Trockne eingeengt. Man erhält 27,4 g (79,3% d. Th.) N-(3'-Phthalsäureanhydrid)-3,4-dihydronaphthalin-1,2-dicarboximid.

Elementaranalyse:

| | | | |
|---|---|---|---|
| berechnet | C 69,57% | H 3,21% | N 4,06% |
| gefunden | C 65,44% | H 3,20% | N 4,44%. |

Beispiel 8

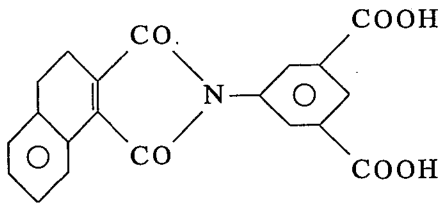

22,6 g 3-Aminoisophthalsäure-dinatriumsalz, 20,0 g 3,4-Dihydronaphthalin-1,2-dicarbonsäureanhydrid und 100 ml N,N-Dimethylacetamid werden zusammengegeben und unter stetigem Rühren während 2 Stunden am Rückfluß gekocht. Dann wird die Reaktionslösung bei einer Temperatur von 80 C mit 220 ml 1 N HCl angesäuert. Der resultierende Niederschlag wird nach erfolgtem Abkühlen auf Raumtemperatur abfiltriert. Das Rohprodukt wird bei 100°C am Vakuum getrocknet. Man erhält 11,9 g (65,6% d Th ) N (4'-Isophthalsäure)-3,4-dihydronaphthalin-1,2-dicarboximid.

Elementaranalyse:

| | | | |
|---|---|---|---|
| berechnet | C 66,12% | H 3,61% | N 3,86% |
| gefunden | C 65,34% | H 3,80% | N 3,80%. |

Beispiel 9

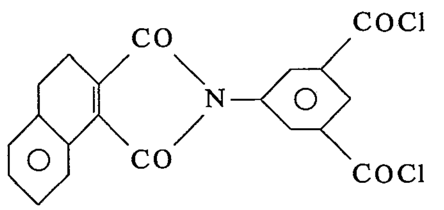

36,3 g (0,1 Mol) des gemäß Beispiel 8 erhaltenen N-(4'-Isophthalsäure)-3,4-dihydronaphthalin-1,2-dicarboximids werden zusammen mit 300 ml Thionylchlorid so lange am Rückfluß gekocht, bis eine klare Lösung vorliegt. Zum Katalysieren der Reaktion werden ca. 5 Tropfen Pyridin zugegeben. Anschließend wird das Reaktionsprodukt am Rotationsverdampfer zur Trockne eingedampft, wobei ein orangeroter Rückstand erhalten wird. Man erhält 36,7 g (86,5% d. Th.) N-(4'-Isophthaloylchlorid)-3,4-dihydronaphthalin-1,2-dicarboximid.

Elementaranalyse (nach dem Umkristallisieren aus Cyclohexan):

| | | | | |
|---|---|---|---|---|
| berechnet | C 60,02% | H 2,77% | N 3,50% | Cl 17,72% |
| gefunden | C 60,21% | H 2,71% | N 3,47% | Cl 17,73% |

Beispiel 10

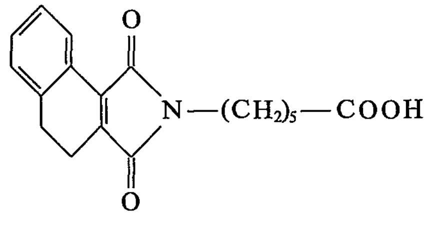

Eine Lösung von 20 g (0,1 Mol) 3,4-Dihydronaphthalin-1,2-dicarbonsäureanhydrid und 13,1 g

(0,1 Mol) 6 Aminocapronsäure in 130 ml Essigsäure wird 6 Std. am Rückfluß gekocht. Die Reaktionslösung wird eingedampft. Das gelbe feste Produkt mit einem Fp. von 109–111 C wird aus 100 ml Tetrachlorkohlenstoff umkristallisiert.

Ausbeute: 23,9 (76,2% d. Th.) Fp. 108–111 C gelb krist.

Analyse:

| | | | |
|---|---|---|---|
| berechnet | C 69,00% | H 6,11% | N 4,47% |
| gefunden | C 68,78% | H 6,12% | N 4,69%. |

NMR Spektrum ($DMSOCH_6$): =7,9 [1H]; 7,15 [3H]; 3,40 [2H, t], 3,00 [2H, t]; 2,60 [2H, t]; 2,20 [2H, t], 1,8–1,1 [6H, Mp].

Beispiel 11

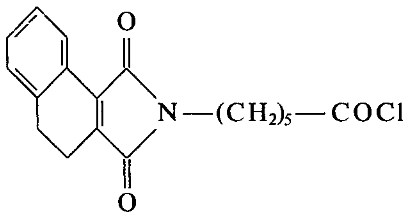

2 g (0,0062 Mol) des gemäß Beispiel 10 erhaltenen N-(capronsäure) 3,4-dihydronaphthalin 1,2 di carboximids und 0,51 ml (0,007 Mol) Thionylchlorid werden in 10 ml Dichlormethan gelöst und 24 Std **bei Raumtemperatur gerührt. Danach wird für 3 Std. zum Rückfluß erhitzt und danach zur Trockne eingedampft. Man erhält 2 g (94,4% d. Th.) öliges Produkt, welches nach 24 Std. kristallisiert. Fp. 65–67°C.**

Analyse:

| | | | | |
|---|---|---|---|---|
| berechnet | C 65,16% | H 5,47% | N 4,22% | Cl 10,69% |
| gefunden | C 65,34% | H 5,54% | N 4,31% | Cl 9,51%. |

Beispiel 12

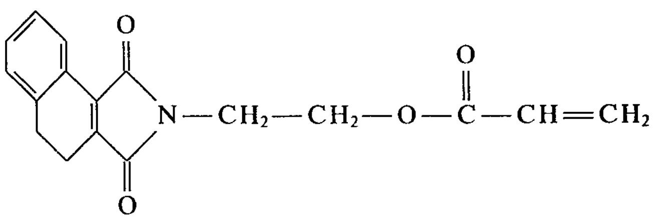

Eine Lösung von 60,82 g N-(2 Hydroxyäthyl) 3,4-dihydronaphthalin-1,2-dicarbonsäureimid, 24,51 g (0,34 Mol) Acrylsäure und 5 ml $H_2SO_4$ chem. rein in 240 ml Toluol werden unter Zusatz von 1,2 g Cu II acetat 1½ Std am Rückfluß gekocht, wobei das entstehende Wasser (4,5 ml) durch einen Wasserabscheider abgetrennt wird.

Die auf Raumtemperatur abgekühlte Lösung wird mit 300 ml 8%iger $NaHCO_3$-Lösung neutralisiert. Die wäßrige Phase wird mit 2 × 400 ml Toluol extrahiert. Die organische Phase wird mit 100 ml Wasser gewaschen, getrocknet und eingedampft.

Ausbeute 53,3 g = 71,65%, Fp. 86–89 C

Analyse

| | | | |
|---|---|---|---|
| berechnet | C 68,68% | H 5,09% | N 4,71% |
| gefunden | C 68,36% | H 5,1% | N 4,74% |

Beispiel 13

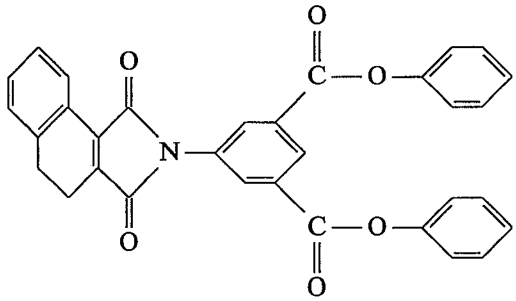

9,5 g (0,100 Mol) Phenol werden in 500 ml wasserfreiem Toluol gelöst und zum Rückfluß erhitzt. 55 ml Toluol werden abdestilliert (Trocknung des Phenols) und auf Raumtemperatur abgekühlt. Bei Raumtemperatur werden 20 g (0,05 Mol) N-(4'-Isophthalsäuredichlorid)-3,4-dihydronaphthalin-1,2-dicarbonimids und 10,36 g Triäthylamin zugegeben und 50 Std. bei Raumtemperatur gerührt. Die starke Suspension wird mit 200 ml Toluol verdünnt und genutscht. Das gelbe Filtrat wird eingedampft und aus 70 ml Äthylenglycolmonomethyläther umkristallisiert.

Ausbeute: 9 g = 35% d. Th., Fp. 205—208° C.

Analyse:

| | | | |
|---|---|---|---|
| berechnet | C 74,56% | H 4,11% | N 2,72% |
| gefunden | C 74,41% | H 3,95% | N 2,78%. |

Beispiel 14

16,25 g (0,077 Mol) 3-Aminoisophthalsäure-di-Na-Salz, 150 ml Wasser, 150 ml Dimethylacetamid und 16,48 g (0,077 Mol) 6,7-Dihydro-5H-benzocyclohepten-8,9-dicarbonsäureanhydrid werden unter Rühren auf 100° erwärmt. Die klare Lösung wird 1 Stunde bei 100° gerührt. Nach dem Abkühlen auf 80° werden 85 ml 2n HCl Lösung zugetropft. Die auf Raumtemperatur gekühlte gelbe Suspension wird abgenutscht und das Nutschgut mit 100 ml kaltem Wasser gewaschen und 12 Stunden im Vakuum bei 100° getrocknet. Man erhält 28 g (96,7% d. Th.) N-(3-Isophthaloyl-dicarbonsäure)-6,7-dihydrobenzocyclohepten-8,9-dicarbonsäureimid mit der folgenden Elementaranalyse für die Summenformel $C_{21}H_{15}NO_6$

| | | | |
|---|---|---|---|
| berechnet | C 66,84% | H 4,01% | N 3,71% |
| gefunden | C 65,8% | H 3,97% | N 3,60%. |

Beispiel 15

5 g (0,013 Mol) N-(3-Isophtholoyldicarbonsäure)-6,7-dihydrobenzocyclohepten-8,9-dicarbonsäureimid, 2 Tropfen Dimethylformamid und 26 ml Thionylchlorid werden 30 Minuten rückflußiert. Die rote Lösung wird eingedampft und das rote kristalline Produkt aus 30 ml trockenem Toluol umkristallisiert. Man erhält 2,4 g (43,7% d. Th.) N-(3-Isophthaloyldicarbonsäurechlorid)-6,7-dihydrobenzocyclohepten-8,9-dicarbonsäureimid mit einem Fp. von 178—181° und folgender Elementaranalyse berechnet für die Summenformel $C_{21}H_{13}NO_4Cl_2$

| | | | | |
|---|---|---|---|---|
| berechnet | C 60,89% | H 3,17% | N 3,38% | Cl 17,12% |
| gefunden | C 60,75% | H 3,21% | N 3,59% | Cl 16,9%. |

Beispiel 16

100 g Copolymerisat aus Methylvinyläther und Maleinsäureanhydrid (1 : 1; Anhydridgehalt = 0,64 Mol, Durchschnittsmolekulargewicht 740 000), 77,8 g (0,32 Mol) des gemäß Beispiel 1 hergestellten N-(2'-Hydroxyäthyl)-3,4-dihydronaphthalin-1,2-dicarboximids und 10 ml Pyridin werden in 1820 ml getrocknetem Tetrahydrofuran gelöst. Das Reaktionsgemisch wird unter Rühren während 72 Stunden bei 80° C gehalten. Nach dem Abkühlen auf Raumtemperatur wird die klare Lösung in 5 Liter Diäthyläther ausgefällt und am Vakuum getrocknet. Man erhält 141,0 g (79,3% d. Th.) eines weißen Polymers. Für die Elementaranalyse wird eine Probe des Polymers in 0,1 N HCl ausgefällt

Elementaranalyse:
gefunden C 55,3% H 5,9% N 2,23%.

Beispiel 17—21

Auf analoge Weise wie im Beispiel 16 beschrieben werden weitere vernetzbare Polymere hergestellt unter Verwendung von:

100 g Copolymerisat gemäß Beispiel 16 und 82,3 g des Imids gemäß Beispiel 2;
100 g Copolymerisat gemäß Beispiel 16 und 82,9 g N-(2'-Hydroxyäthyl)-2-methyl-2H-chromen-3,4-dicarboximid;
100 g Copolymerisat gemäß Beispiel 16 und 78,4 g N-(2'-Hydroxyäthyl)-2,3-dihydro-1-benzoxepin-4,5-dicarboximid;
100 g Copolymerisat gemäß Beispiel 16 und 87,4 g N-(3'-Hydroxy-2',2'-dimethylpropyl)-3,4-dihydronaphthalin-1,2-dicarbonsäureimid;
100 g Copolymerisat gemäß Beispiel 16 und 95,6 g N-(3'-Hydroxy-2',2'-dimethylpropyl)-6,7-dihydro-5H-benzocyclohepten-8,9-dicarbonsäureimid.

Beispiel 22

31,1 g (0,1 Mol) N-(2'-Methacryloyloxyäthyl)-3,4-dihydronaphthalin-1,2-dicarbonsäureimid werden zusammen mit 0,31 g α,α'-Azoisobutyronitril in 140 ml Tetrahydrofuran gelöst. Das Reaktionsgemisch wird unter Stickstoffatmosphäre und ständigem Umrühren bei leichtem Rückfluß (ca. 80° C) während 6 Stunden polymerisiert. Nach beendeter Reaktion wird auf Raumtemperatur abgekühlt, und das Polymere wird durch Eintropfen der Reaktionslösung in 2 Liter Hexan ausgefällt. Man erhält 24,4 g (78,5% d. Th.) eines weißen Pulvers; inhärente Viskosität: 0,15 dl/g (0,5 Gew.-% in N,N-Dimethylformamid bei 20° C).

Beispiel 23

2,5 g (0,00662 Mol) N-(3-Isophthaloyldicarbonsäure)-6,7-dihydrobenzocyclohepten-8,9-dicarbonsäureimid und 1,75 g (0,00729 Mol) 1,3-Diglycidyl-5,5-dimethylhydantoin werden in 85 ml Cyclohexanon gelöst und mit 1 Kristall Tetrabutylammoniumchlorid versetzt. Die Lösung wird 2 Std. bei 110° gerührt. Die gelbe, leicht viskose Lösung kann direkt zur Beschichtung von Kupferplatten verwendet werden.

Beispiel 24

In einem 750 ml Sulfierkolben werden 7,3 g (0,0639 Mol) 2,5-Dimethylpiperazin und 18 ml Triäthylamin in 100 ml trockenem Chloroform gelöst und auf −5° gekühlt. Bei dieser Temperatur wird eine Suspension von 8 g (0,0193 Mol) N-(3-Isophthaloyldicarbonsäurechlorid)-6,7-dihydrobenzocyclohepten-8,9-dicarbonsäureimid und 10,78 g (0,04506 Mol) Sebacinsäuredichlorid in 100 ml Chloroform bei −5° zugetropft. Bei Raumtemperatur wird 3 Stunden ausgerührt und die leicht viskose Lösung bei 1500 ml Petroläther ausgefällt. Man erhält 13 g beiges Polymer.

Beispiel 25

Dieses Beispiel betrifft Abbildungen, die durch Photovernetzung von mit erfindungsgemäßen Verbindungen hergestellten Polymeren erzeugt und durch Anfärben besser sichtbar gemacht werden, sowie die Ermittlung der relativen Empfindlichkeit der so erhaltenen Abbildungen. Belichtet wird mit einer 400 Watt Quecksilberdampf-Hochdrucklampe in einem Abstand von 40 cm zum Vakuumtisch. Als Vorlage dient ein Stauffer-Stufenkeil wie beschrieben in »Photoresist, Material and Processes«, W. S. De Forest, S. 110 (McGraw-Hill Book Company, New York, 1975).

Beschichtung: Das lichtvernetzbare Polymere wird durch Aufschleudern mit 1000 Touren/Minute aus einer 5%igen Lösung in N,N-Dimethylformamid auf Aluminiumplatten (ca. 0,3 mm) aufgetragen.

Entwicklung: 3 Sekunden in Tetrahydrofuran; 30 Sekunden in 3%iger $NaHCO_3 \cdot H_2O$.

Einfärbung: Das bildmäßig vernetzte Polymere läßt sich anschließend leicht mit einem kationischen

Farbstoff anfärben, z. B. durch 30 Sekunden Einfärben in einer 5%igen wäßrigen Lösung des Farbstoffs der Formel

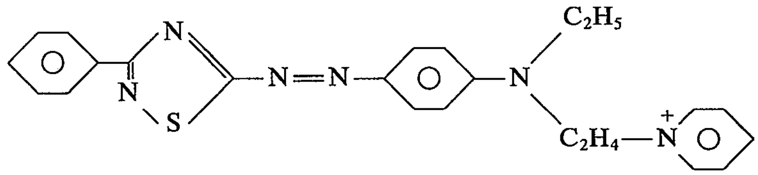

In der folgenden Tabelle ist die Anzahl angefärbter Stufen im Stufenkeil bei entsprechender Belichtungszeit angegeben.

| Polymer gemäß | Belichtungszeit | Anzahl angefärbter Stufen im Stufenkeil |
|---|---|---|
| Beispiel 16 | 6 Minuten | 9 |
| | 3 Minuten | 7 |
| | 1 Minute | 5 |
| Beispiel 17 | 9 Minuten | 5 |
| | 6 Minuten | 2 |
| | 3 Minuten | 1 |
| Beispiel 18 | 9 Minuten | 5 |
| | 6 Minuten | 3 |
| | 3 Minuten | 1 |
| Beispiel 19 | 9 Minuten | 7 |
| | 6 Minuten | 5 |
| | 3 Minuten | 3 |

**Patentansprüche**

1. Eine Verbindung der Formel I

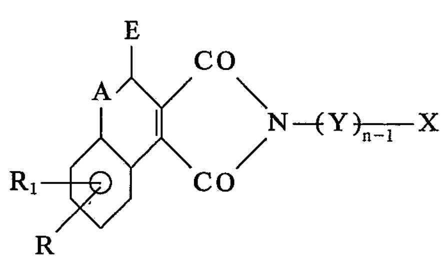

(I)

worin n die Zahl 1 oder 2, R und $R_1$ unabhängig voneinander Wasserstoff, Halogen, Alkyl mit 1—4 C-Atomen oder Methoxy, A $-CH_2-$, $-CH_2CH_2-$ oder $-OCH_2-$ mit an den aromatischen Ring gebundenem Sauerstoffatom und E Wasserstoff oder A $-O-$ und E $-CH_3$ und Y gegebenenfalls durch Heteroatome unterbrochenes Alkylen mit 1—30 C-Atomen, Cycloalkylen mit 5 oder 6 C-Atomen, einen Dicyclohexylmethanrest, Arylen mit 6—10 C-Atomen, Aralkylen oder Alkylarylen mit 7 oder 8 C-Atomen bedeuten, wobei die genannten Reste Y mit Alkyl- oder Alkoxygruppen mit je 1—4

C-Atomen, Nitrogruppen oder Halogenatomen substituiert sein können, X, bei n = 1, eine Gruppe der Formeln

—NH—CO-Alkenyl oder 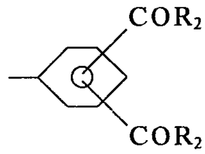

und bei n = 2, $-OH$, $-NH_2$, $-NH$-Alkyl mit 1—4 C-Atomen, $-SH$, $-COOH$, $-COCl$, $-CO-O$-Alkenyl, $-O$-Alkenyl, $-O-CO$-Alkenyl, $-NH-CO$-Alkenyl oder $-S-CO$-Alkenyl darstellt, die beiden $-COR_2$ in meta- oder para-Stellung zueinander an den Benzolring gebunden sind und die $R_2$ je $-OH$, $-Cl$, Alkoxy mit 1—4 C-Atomen oder Phenoxy bedeuten oder die beiden $-COR_2$ in ortho-Stellung zueinander an den Benzolring gebunden sind und eines von $R_2$ $-OH$ oder $-O^-M^+$ und das andere

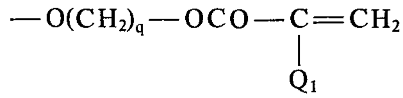

oder beide $R_2$ zusammen $-O-$ bedeuten, $M^+$ ein Alkalimetallkation, ein Pyridiniumkation oder ein Trialkylammoniumkation mit 3—24 C-Atomen, $Q_1$ Wasserstoff oder Methyl und q eine ganze Zahl von 2—4 bedeuten, wobei in den obigen Gruppen Alkenylteile 2—4 C-Atome aufweisen und A bei n = 2 und $Y = -CH_2-$ einen von $-CH_2-$ verschiedenen Rest darstellt.

2. Eine Verbindung der Formel I nach Anspruch 1, worin R und $R_1$ je Wasserstoff, A $-CH_2-$, $-CH_2CH_2-$ oder $-OCH_2-$ mit an den aromatischen Ring gebundenem Sauerstoffatom, E Wasserstoff, Y eine geradkettige oder verzweigte Alkylengruppe mit 2—11 C-Atomen, eine 1,3- oder 1,4-Phenylengruppe oder die 1,4-Cyclohexylengruppe, X, bei n = 1, eine Gruppe der Formeln

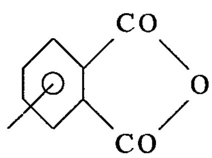 oder 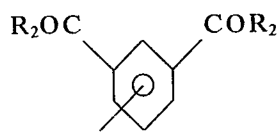

worin die beiden $R_2$ je $-OH$, $-Cl$, Methoxy, Äthoxy oder Phenoxy bedeuten, und X, bei n = 2, $-OH$, $-NH_2$, $-COOH$, $-COCl$, $-COO$-Alkenyl, $-O$-Alkenyl oder $-O-CO$-Alkenyl darstellen und die Alkenylteile in den genannten Substituenten 2—4 C-Atome aufweisen.

3. Eine Verbindung der Formel I nach Anspruch 2, worin A $-CH_2-$ und E Wasserstoff bedeuten.

4. Eine Verbindung nach Anspruch 1, dadurch gekennzeichnet, daß sie der Formel X

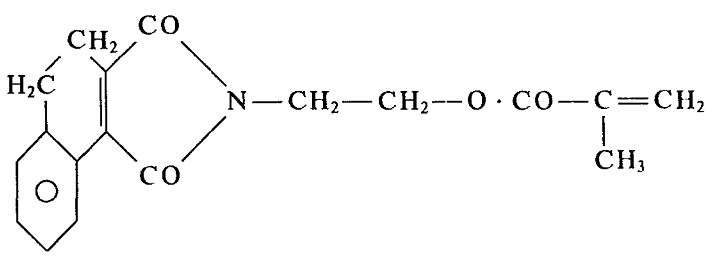 (X)

entspricht.

5. Eine Verbindung nach Anspruch 1, dadurch gekennzeichnet, daß sie der Formel XI

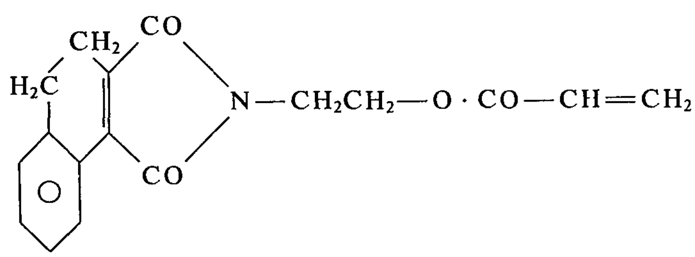 (XI)

entspricht.

6. Eine Verbindung nach Anspruch 1, dadurch gekennzeichnet, daß sie der Formel

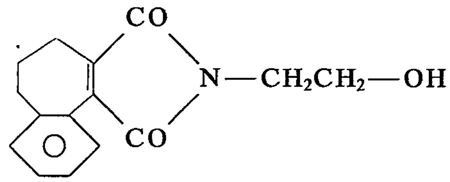

(XII)

entspricht.

7. Eine Verbindung nach Anspruch 1, dadurch gekennzeichnet, daß sie der Formel XIII

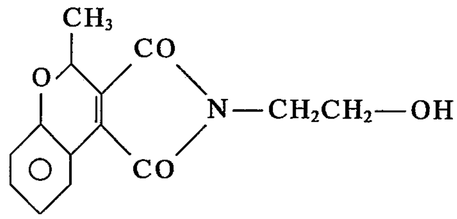

(XIII)

entspricht.

8. Eine Verbindung nach Anspruch 1, dadurch gekennzeichnet, daß sie der Formel XIV

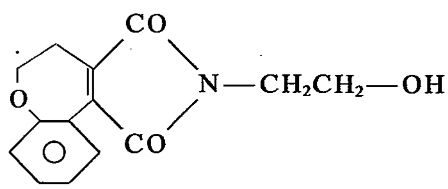

(XIV)

entspricht.

9. Ein Verfahren zur Herstellung einer Verbindung nach Anspruch 1 der Formel Ia oder Ib

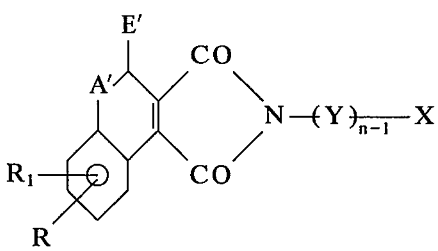

oder

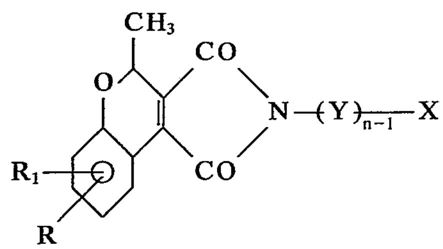

(Ia) (Ib)

worin A' $-CH_2$, $-CH_2CH_2-$ oder $-OCH_2-$ mit an den aromatischen Ring gebundenem Sauerstoffatom und E' Wasserstoff bedeuten, R, $R_1$, X, Y und n die unter Formel I angegebene Bedeutung haben und A' bei $n=2$ und $Y=-CH_2-$ einen von $-CH_2-$ verschiedenen Rest bedeutet, dadurch gekennzeichnet, daß man entweder (a) eine Verbindung der Formel II

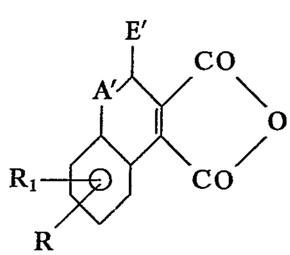

(II)

mit einer Verbindung der Formel III

$$H_2N-(Y)_{n-1}-X' \qquad (III)$$

umsetzt, wobei A' und E' die unter Formel Ia angegebene und R, $R_1$, Y und n die unter Formel I

angegebene Bedeutung haben und X', bei n = 1, eine Gruppe der Formel

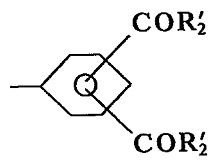

,

worin die $-COR_2'$-Gruppen in meta- oder para-Stellung zueinander an den Benzolring gebunden sind und die $R_2$ je $-OH$, $-O^-M^+$, Alkoxy mit 1—4 C-Atomen oder Phenoxy bedeuten oder worin die $-COR_2'$-Gruppen in ortho-Stellung zueinander an den Benzolring gebunden sind und die beiden $R_2'$ zusammen $-O-$ darstellen, $M^+$ die unter Formel I angegebene Bedeutung hat, und, bei n = 2, X' $-OH$, $-NH_2$, —NH-Alkyl mit 1—4 C-Atomen, $-COOH$, —CH oder —O-Alkenyl mit 2—4 C-Atomen im Alkenylteil bedeutet, gegebenenfalls intermediär gebildete Amidcarbonsäure cyclisiert und das Imid anschließend gegebenenfalls in eine Verbindung der Formel Ia überführt, worin X eine von X' verschiedene Bedeutung hat, oder (b) eine Verbindung der Formel I, worin A $-OCH_2-$ mit an den aromatischen Ring gebundenem Sauerstoffatom darstellt und R, $R_1$, X, Y und n die unter Formel I angegebene Bedeutung haben, thermisch oder in Gegenwart eines basischen Katalysators in eine Verbindung der Formel Ib umlagert.

10. Ein Verfahren nach Anspruch 9, dadurch gekennzeichnet, daß man eine Verbindung der in einem der Ansprüche 1—3 genannten Art herstellt.

## Claims:

1. A compound of the formula I

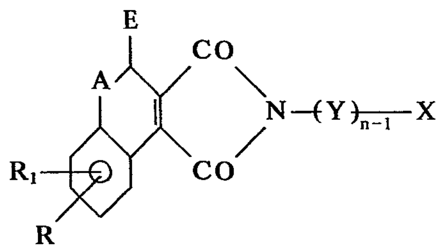

(I)

in which n is 1 or 2, each of R and $R_1$ independently is hydrogen, halogen, $C_1$—$C_4$alkyl or methoxy, A is $-CH_2-$, $-CH_2CH_2-$ or $-OCH_2-$ with the oxygen atom attached to the aromatic ring and E is hydrogen or A is $-O-$ and E is $-CH_3$, and Y is $C_1$—$C_{30}$alkylene which can be interrupted by hetero-atoms, or is $C_5$—$C_6$cycloalkylene, a dicyclohexylmethane radical, $C_6$—$C_{10}$arylene, or $C_7$—$C_8$aralkylene or alkylarylene, while said radicals Y can be substituted by alkyl or alkoxy groups, each of 1 or 4 carbon atoms, nitro groups or halogen atoms, and X, when n = 1, is a group of the formula —NH—CO-alkenyl or

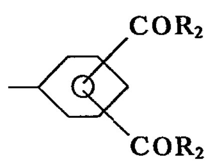

and, when n = 2, is $-OH$, $-NH_2$, —NH-alkyl having 1—4 C atoms, $-SH$, $-COOH$, $-COCl$, —CO—O-alkenyl, —O-alkenyl, —O—CO-alkenyl, —NH—CO-alkenyl or —S—CO-alkenyl, the two $-COR_2$s are attached to the benzene ring in the meta- or para-position to each other and each $R_2$ is $-OH$, $-Cl$, $C_1$—$C_4$alkoxy or phenoxy, or the two $-COR_2$s are attached to the benzene ring in the ortho-position to each other and one $R_2$ is $-OH$ or $-O^-M^+$ and the other is

$$-O(CH_2)_q-OCO-\underset{\displaystyle Q_1}{\underset{|}{C}}=CH_2$$

or both $R_2$s together are $-O-$, and $M^+$ is an alkali metal cation, a pyridinium cation or a trialkylammonium cation having 3—24 C atoms, $Q_1$ is hydrogen or methyl and q is an integer from 2 to 4,

and alkenyl moieties in the above groups have 2—4 C atoms and, when n=2 and Y=$-CH_2-$, A is a radical which differs from $-CH_2-$.

2. A compound of the formula I according to claim 1, in which each of R and $R_1$ is hydrogen, A is $-CH_2-$, $-CH_2CH_2-$ or $-OCH_2-$ with the oxygen atom attached to the aromatic ring, E is hydrogen, Y is a straight-chain or branched $C_2$—$C_{11}$alkylene group, a 1,3- or 1,4-phenylene group or the 1,4-cyclohexylene group and X, when n=1, is a group of the formula

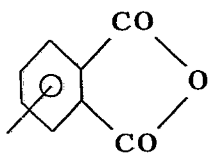
or
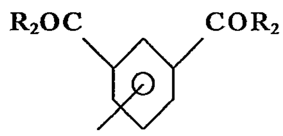

in which each $R_2$ is $-OH$, $-Cl$, methoxy, ethoxy or phenoxy, and, when n=2, is $-OH$, $-NH_2$, $-COOH$, $-COCl$, $-COO$-alkenyl, $-O$-alkenyl or $-O-CO$-alkenyl, and the alkenyl moieties in the said substituents contain 2—4 C atoms.

3. A compound of the formula I according to claim 2, in which A is $-CH_2-$ and E is hydrogen.

4. A compound according to claim 1 which has the formula X

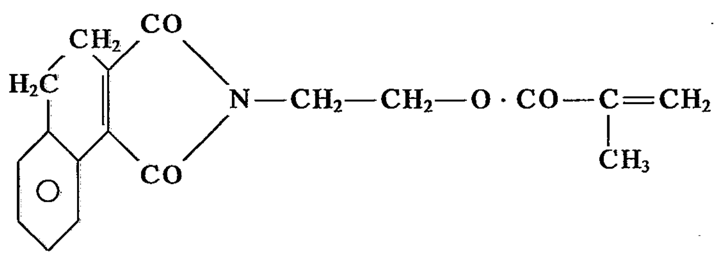
(X)

5. A compound according to claim 1 which has the formula XI

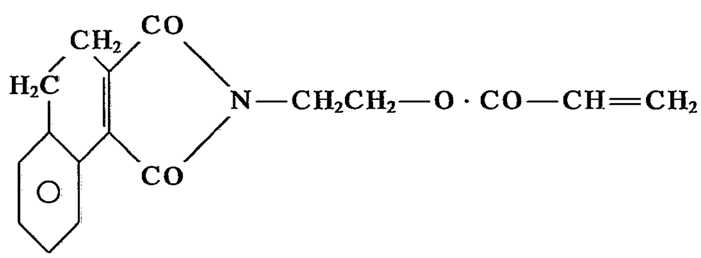
(XI)

6. A compound according to claim 1 which the formula

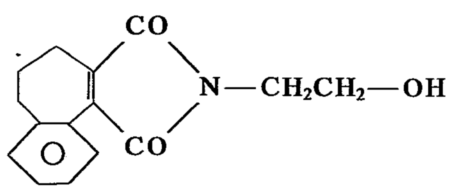
(XII)

7. A compound according to claim 1 which has the formula XIII

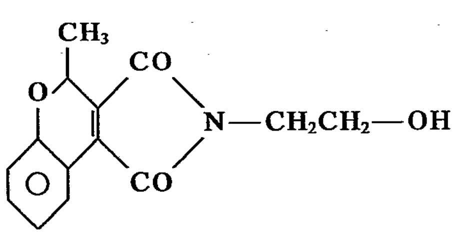
(XIII)

8. A compound according to claim 1 which has the formula XIV

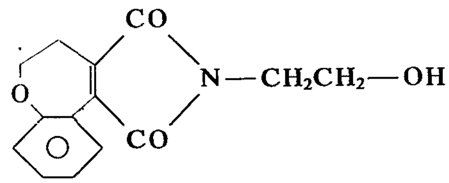

(XIV)

9. A process for the preparation of a compound according to claim 1 of the formula Ia or Ib

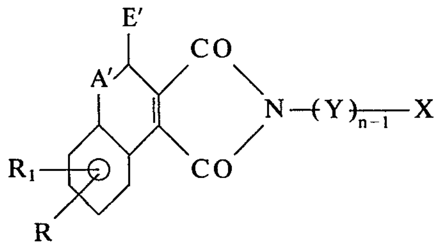

(Ia)

or

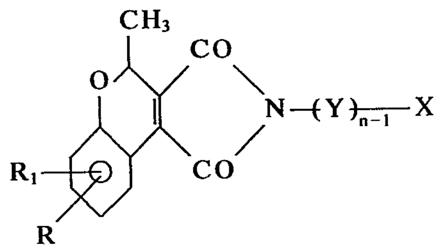

(Ib)

in which A′ is $-CH_2-$, $-CH_2CH_2-$ or $-OCH_2-$ with the oxygen atom attached to the aromatic ring and E′ is hydrogen, R, $R_1$, X, Y and n are as defined for formula I and, when $n = 2$ and $Y = -CH_2-$, A′ is a radical which differs from $-CH_2-$, characterised in that eiter (a) a compound of the formula II

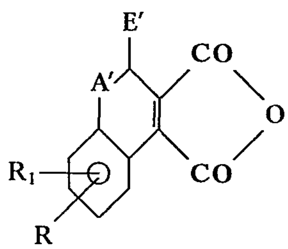

(II)

is reacted with a compound of the formula III

$$H_2N{-}(Y)_{n-1}{-}X' \qquad (III)$$

in which formulae A′ and E′ are as defined for formula Ia and R, $R_1$, Y and n are as defined for formula I and X′, when $n = 1$, is a group of the formula

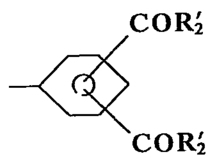

in which the $-COR_2'$ groups are attached to the benzene ring in the meta- or para-position to each other and each $R_2$ is $-OH$, $-O^-\ M^+$, $C_1-C_4$alkoxy or phenoxy, or in which the $-COR_2'$ groups are attached to the benzene ring in the ortho-position to each other and both $R_2$s together are $-O-$, $M^+$ is as defined for formula I, and, when $n = 2$, is $-OH$, $-NH_2$, $-NH$-alkyl having 1-4 C atoms, $-COOH$, $-SH$ or $-O$-alkenyl containing 2-4 C atoms in the alkyl moiety, if necessary amidocarboxylic acids obtained as intermediates are cyclised and then, if desired, the imide is converted into a compound of the formula Ia in which X differs from X′, or (b) a compound of the formula I, in which A is $-OCH_2-$ whith the oxygen atom bonded to the aromatic ring and R, $R_1$, X, Y and n are as defined for formula I, is rearranged to a compound of the formula Ib by the action of heat or in the presoence of a basic catalyst.

10. A process according to claim 9, characterised in that a compound of the type indicated in any one of claims 1-3 is prepared.

**Revendications:**

1. Composé répondant à la formule I

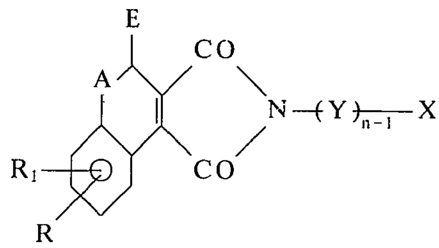

(I)

dans laquelle n désigne le nombre 1 ou 2, R et $R_1$ représentent chacun, indépendamment l'un de l'autre, l'hydrogène, un halogène, un alkyle en $C_1-C_4$ ou un méthoxy, A représente $-CH_2-$, $-CH_2CH_2-$ ou $-OCH_2-$, l'atome d'oxygène de ce dernier étant relié au noyau aromatique, et E représente l'hydrogène, ou A représente $-O-$ et E $-CH_3$, et Y représente un radical alkylène en $C_1-C_{30}$ éventuellement interrompu par des hétéroatomes, un cycloalkylène en $C_5$ ou $C_6$, un radical dicyclohexyl-méthane, un arylène en $C_6-C_{10}$, un radical aralkylène ou alkylarylène en $C_7$ ou $C_8$, les radicaux Y mentionnés pouvant porter des substituants pris dans l'ensemble constitué par les radicaux alkyles et alcoxy en $C_1-C_4$, le groupe nitro et les atomes d'halogènes, X représente, dans le cas où n est égal à 1, un radical $-NH-CO$-alcényle ou un radical de formule

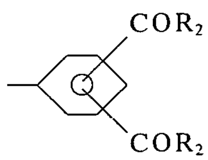

et, dans le cas où n est égal à 2, $-OH$, $-NH_2$, $-NH$-alkyle en $C_1-C_4$, $-SH$, $-COOH$, $-COCl$, $-COO$-alcényle, $-O$-alcényle, $-O-CO$-alcényle, $-NH-CO$-alcényle ou $-S-CO$-alcényle, les deux groupes $-COR_2$ étant en position méta ou para l'un par rapport à l'autre sur le noyau benzénique et les $R_2$ représentant chacun $-OH$, $-Cl$, un alcoxy en $C_1-C_4$ ou un phénoxy, ou les deux groupes $-COR_2$ étant en position ortho l'un par rapport à l'autre sur le noyau benzénique et l'un des $R_2$ représentant $-OH$ ou $-O^- M^+$ et l'autre

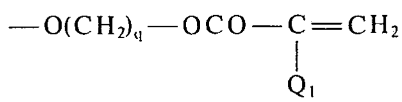

ou les deux $R_2$ formant ensemble $-O-$, $M^+$ représente un cation de métal alcalin, un cation pyridinium ou un cation trialkylammonium contenant de 3 à 24 atomes de carbone, $Q_1$ représente l'hydrogène ou un méthyle et q un nombre entier de 2 à 4, les parties alcényliques présentes dans les groupes mentionnés ci-dessus contenant de 2 à 4 atomes de carbone, et, dans le cas où n est égal à 2 et où Y désigne un radical $-CH_2-$, A n'est pas un radical $-CH_2-$.

2. Composé de formule I selon la revendication 1, dans lequel R et $R_1$ représentent chacun l'hydrogène, A représente $-CH_2-$, $-CH_2CH_2-$ ou un radical $-OCH_2-$ relié par son atome d'oxygène au noyau aromatique, E représente l'hydrogène, Y représente un radical alkylène en $C_2-C_{11}$, linéaire ou ramifié, un radical phénylène-1,3 ou -1,4 où un radical cyclohexylène-1,4, X représente, dans le cas où n est égal à 1, un radical répondant à la formule:

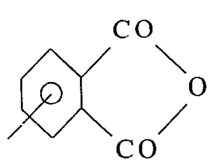

ou à la formule:

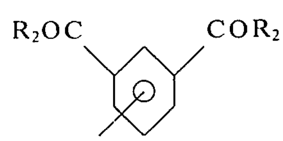

dans laquelle les deux $R_2$ représentent chacun $-OH$, $-Cl$, un méthoxy, un éthoxy ou un phénoxy, et, dans le cas où n est égal à 2, X représente $-OH$, $-NH_2$, $-COOH$, $-COCl$, $-COO$-alcényle, **$-O$-alcényle ou $-O-CO$-alcényle, les parties alcényliques dans les substituants mentionnés contenant de 2 à 4 atomes de carbone.**

3. Composé de formule I selon la revendication 2, dans lequel A représente $-CH_2-$ et E de l'hydrogène.

4. Composé selon la revendication 1, caractérisé en ce qu'il répond à la formule X

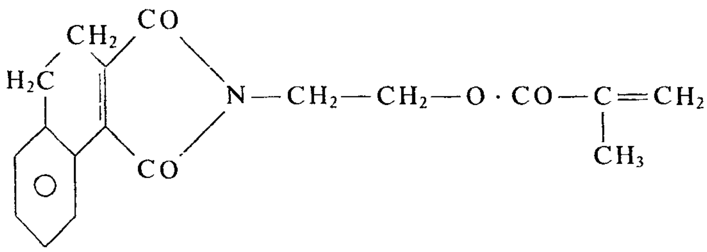

(X)

5 Composé selon la revendication 1, caractérisé en ce qu'il répond à la formule XI

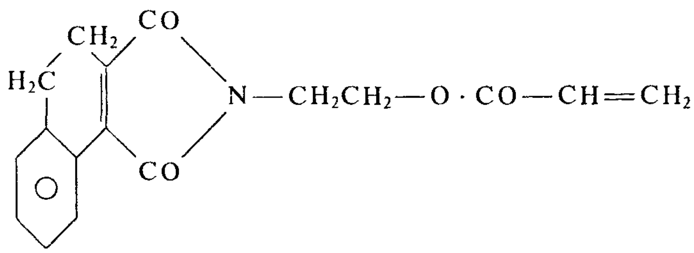

(XI)

6 Composé selon la revendication 1, caractérisé en ce qu'il répond à la formule XII

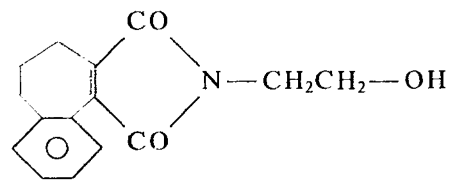

(XII)

7 Composé selon la revendication 1, caractérisé en ce qu'il répond a la formule XIII

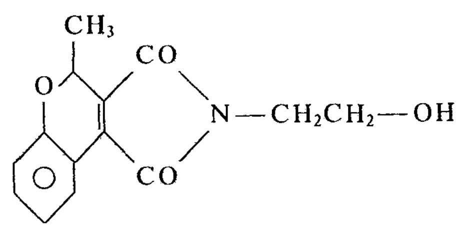

(XIII)

8 Compose selon la revendication 1, caractérisé en ce qu'il répond à la formule XIV

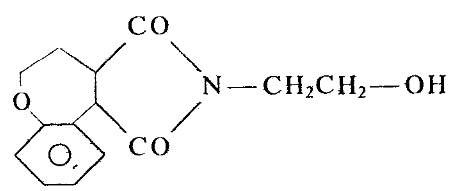

(XIV)

9. Procédé de préparation d'un composé selon la revendication 1 qui répond à l'une des formules Ia et Ib

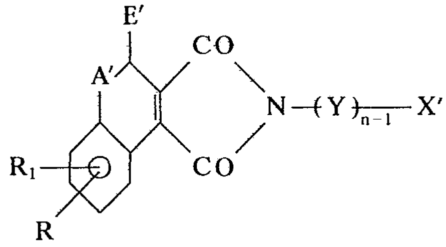

(Ia)

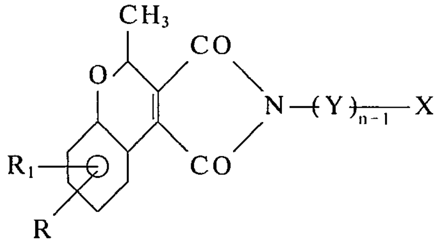

(Ib)

dans lesquelles A représente $-CH_2-$, $-CH_2CH_2-$ ou un radical $-OCH_2-$ relié par son atome d'oxygène au noyau aromatique, E' représente l'hydrogène, R, $R_1$ X, Y et n ont les significations données sous la formule I et A', lorsque n est égal à 2 et que Y est un radical $-CH_2-$, ne représente pas un radical $-CH_2-$, procédé caractérisé en ce que ou bien (a) on fait réagir un composé de formule II

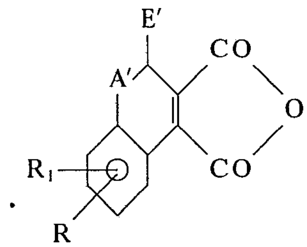

(II)

avec un composé de formule III

$$H_2N-(Y)_{\overline{n-1}}-X' \qquad (III)$$

formules dans lesquelles A' et E' ont les significations données sous la fourmule Ia, R, $R_1$, Y et n ont les significations données sous la formule I et X' représente, dan les cas où n est égal à 1, un radical de formule

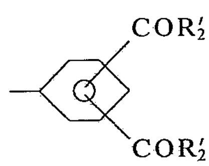

dans lequel les groupes $-COR'_2$ sont en position méta ou para l'un par rapport à l'autre sur le noyau benzénique et les $R'_2$ représentent chacun $-OH$, $-O^-M^+$, un alcoxy en $C_1-C_4$ ou un phénoxy, ou dans lequel les groupes $-COR'_2$ sont en position ortho l'un par rapport à l'autre sur le noyau benzénique et les deux $R'_2$ représentent ensemble $-O-$, et $M^+$ a la signification donnée sous la formule I, et, dans le cas où n est égal à 2, X' représente $-OH$, $-NH_2$, $-NH$-alkyle en $C_1-C_4$, $-COOH$, $-SH$ ou un $-O$ alcényle en $C_2-C_4$, on cyclise les amid e-acides carboxyliques qui se sont éventuellement formés intermédiairement, puis on transforme éventuellement l'imide en un composé de formule Ia dans lequel X a une signification autre que celle de X', ou bien (b) on transpose un composé de formule I dans lequel A représente un radical $-OCH_2-$ relié par son atome d'oxygène au noyau aromatique et R, $R_1$, X, Y et n ont les significations données sous la formule I, par voie thermique ou en présence d'un catalyseur basique, de manière à obtenir un composé de formule Ib.

10 Procédé selon la revendication 9, caractérisé en ce qu'on prépare un composé du type indiqué dans l'une des revendications 1 à 3.